# EUROPEAN PATENT APPLICATION

(11) **EP 2 481 808 A1**
(43) Date of publication of application: **01.08.2012**
(21) Application number: 10818555.4
(22) Date of filing: 22.09.2010
(51) Int. Cl.: C12Q 1/48, C12N 5/10, G01N 33/15, G01N 33/50, G01N 33/68, C12N 15/09

(54) **AMELIORATION OF METABOLIC SYNDROME OR METHOD FOR SCREENING FOR AMELIORATING AGENT FOR METABOLIC SYNDROME UTILIZING PHYSIOLOGICAL FUNCTION OF SPHINGOMYELIN SYNTHASE SMS2**

(30) Priority: 24.09.2009 JP 2009219751
(71) Applicant: National University Corporation Hokkaido University, Sapporo-shi, Hokkaido 060-0808 (JP); Shionogi & Co., Ltd., Osaka 541-0045 (JP)
(72) Inventor: MITSUTAKE, Susumu, Sapporo-shi Hokkaido 060-0808 (JP); IGARASHI, Yasuyuki, Sapporo-shi Hokkaido 060-0808 (JP); ZAMA, Kota, Sapporo-shi, Hokkaido 060-0808 (JP); YOSHIDA, Tetsuya, Sapporo-shi Hokkaido 001-0021 (JP); TANAKA, Yoshikazu, Sapporo-shi Hokkaido 001-0021 (JP); TAKEMOTO, Hiroshi, Sapporo-shi Hokkaido 001-0021 (JP)
(74) Representative: Vossius & Partner
(86) International application number: PCT/JP2010/005751
(87) International publication number: WO 2011/036878

(57) **Abstract**

Disclosed is a method for screening for a medicinal agent that can decrease a body weight, a medicinal agent that can reduce a visceral fat, a medicinal agent that can reduce a triglyceride in the liver, and a medicinal agent that can ameliorate obesity and fatty liver. The method involves a step of measuring the inhibitory activity of a candidate substance on a sphingomyelin synthase, wherein the candidate substance is determined to have at least one function selected from the group consisting of an anti-obesity agent, a visceral fat-reducing agent, a fatty liver-treating agent and an adiponectin expression enhancer when the candidate substance has an inhibitory activity on the sphingomyelin synthase.

## Description

### [Technical Field]

The present invention relates to a method for screening for amelioration of metabolic syndromes available in a pharmaceutical products and functional foods.

### [Background Art]

The induction of obesity has been known to be involved in an action of accelerating eating via proteins that are expressed in the hypothalamus such as neuropeptide Y5 receptor, or melanocortin receptor, an action of accelerating the synthesis of fatty acids via acyl-CoA carboxylase (ACC) or the like. Currently, as an anti-obesity drug, a neuropeptide Y5 receptor antagonist, a melanocortin receptor antagonist and an ACC inhibitor or the like have been developed. However, to date, an effective anti-obesity drug has not been found.

Fatty liver is a disease, which a neutral fat accumulates in the liver, and obesity are deemed as a major cause of developing it. However, similarly to an anti-obesity drug, an effective drug for treating fatty liver has not been found to date. In addition, regarding diabetes which is deemed as a representative example of a metabolic syndrome and also deemed as a fundamental cause of lifestyle related diseases such as obesity or circulatory system diseases, sulfonylurea drugs, biguanides, alpha-glucosidase and azolidine derivatives and the like have been developed. However, problems for side effect and efficacy thereof exist. Therefore, a more effective drug for treatment is expected to be developed.

A sphingomyelin synthetase (SMS) is an enzyme which synthesizes sphingomyelin(SM), a sphingolipid most abundant in a cell membrane, and plays an important role in cell death and survival (see, NPLs 1 and 2). SMS was identified in 2004, and are known to be present as two kinds, SMS1 and SMS2. SMS1 is known to be expressed in the Golgi body and be involved in de novo synthesis of SM. On the other hand, SMS2 is expressed in the Golgi body and cell membrane and is unknown for its physiological function in detail (see, NPL 3), and is merely suggested in NPLs 4-6 for the possibility of involving in arteriosclerosis.

To date, methods for ameliorating a metabolic syndrome by using neutral fat metabolism/absorbance inhibitors or inhibiting agents, agents for accelerating neutral fat metabolism or the like have been attempted. Specifically, these are drugs for ameliorating hyperlipidemia and type II diabetes by synthetic ligands of intranuclear receptor PPARs, drugs for ameliorating hyperlipidemia via inhibiting cholesterol synthesis by statin agents, or the like.

To date, an action of inducing obesity via controlling the synthesis of sphingolipids have not been known. A SMS inhibitor developed as an anti-diabetes drug, an anti-obesity drug or a drug for treating fatty liver does not exist, either.

### [Citation List]

### [Non Patent Literature]

[NPL 1] Yamaoka et al, The Journal of Biological Chemistry,279, 18688-18693, (2004)
[NPL 2] Huitema et al, The EMBO Journal, 23, 33-44, (2004)
[NPL 3] Tafesse et al, The Journal of Biological Chemistry, 281, 29421-29425, (2006)
[NPL 4] Park et al, Circulation, 110, 3465-3471, (2004)
[NPL 5] Liu et al, Arteriosclerosis, Thrombosis, and Vascular Biology, 29, 850-856, (2009)
[NPL 6] Liu et al, Circulation Research, 105, 295-303, (2009)

### [Summary of Invention]

### [Technical Problem]

The present invention relates to decreasing body weight, decreasing visceral fat, decreasing triglycerides in the liver and ameliorating obesity and fatty liver by suppressing the synthesis of sphingolipids. Another aspect of the present invention is related to the provision of a method for screening for a drug for metabolic syndrome such as anti-diabetes, anti-obesity drug and drug for treating fatty liver which ameliorates obesity and fatty liver by inhibiting sphingolipid substrate synthesis. Moreover, another aspect of the present invention is related to a cell used for said screening.

### [Means for solving problem]

With respect to the above-mentioned problems, the authors have achieved the present invention by discovering that controlling synthetic enzyme of sphingomyelin allows treatment and prevention of the above-mentioned diseases. As such, the present invention provides the following:
(Item 1) A method for screening a substance effective for treating or preventing a metabolic syndrome, comprising the step of measuring inhibitory activity of a subject substance against sphingomyelin synthase, wherein when the subject substance has an inhibitory activity of a sphingomyelin synthase, the subject substance is determined to be effective for treating or preventing the metabolic syndrome.
(Item 2) The method according to item 1, wherein the substance effective for treating or preventing the metabolic syndrome comprises at least one function selected from the group consisting of an anti-diabetes, an anti-obesity drug, a drug for decreasing visceral fat, a drug for treating fatty liver and a drug for increasing adiponectin expression.
(Item 3) The method according to item 1 or 2, wherein the measurement of the inhibitory activity is carried out using a cell in which either one gene of sphingomyelin synthase SMS1 or SMS2 is knocked out.
(Item 4) The method according to item 1-3, wherein the measurement of the inhibitory activity is carried out using a cell in which either one gene of sphingomyelin synthase SMS1 or SMS2 is knocked out and has at least one sphingomyelin synthase genetically introduced therein.
(Item 5) The method according to item 3 or 4, wherein when the cell is given cyclodextrin and the subject substance causes delay in cell growth or cause cell death, the subject substance is determined to have said at least one function.
(Item 6) The method according to item 3-5, wherein said at least one sphingomyelin synthase is SMS1 or SMS2, or both SMS1 and SMS2.
(Item 7) The method according to item 3-6, wherein said at least one sphingomyelin synthase is SMS2.
(Item 8) A kit for screening for a substance effective for treating or preventing a metabolic syndrome, comprising a cell in which either one gene of sphingomyelin synthase SMS1 or SMS2 is knocked out.
(Item 9) A kit for screening for a substance effective for treating or preventing a metabolic syndrome, comprising a cell in which either one gene of sphingomyelin synthase SMS1 or SMS2 is knocked out and has at least one sphingomyelin synthase genetically introduced therein.
(Item 10) The kit according to item 8 or 9, wherein said substance comprises at least one function selected from the group consisting of an anti-diabetes, an anti-obesity drug, a drug for decreasing visceral fat, a drug for treating fatty liver and a drug for increasing adiponectin expression
(item 11) The kit according to item 10, further comprising cyclodextrin.
(Item 12) The kit according to any one of items 8-11, wherein said at least one sphingomyelin synthase is SMS1 or SMS2, or both SMS1 and SMS2.
(Item 13) The kit according to any one of items 8-12, wherein said at least one sphingomyelin synthase is SMS2.
(Item 14) A cell for screening for a substance effective for treating or preventing a metabolic syndrome, in which either one gene of sphingomyelin synthase SMS1 or SMS2 is knocked out.
(Item 15) A cell in which sphingomyelin synthase SMS1 and SMS2 have been knocked out wherein at least one sphingomyelin synthase is genetically introduced thereinto.
Alternatively, the present invention provides the following:
(Item A1) A method comprising the step of measuring an inhibitory activity of a candidate substance against sphingomyelin synthase, wherein when the candidate substance has the inhibitory activity for sphingomyelin synthase, the candidate substance is determined to have at least one function selected from the group consisting of an anti-obesity drug, a drug for decreasing visceral fat, a drug for treating fatty liver and a drug for increasing adiponectin expression.
(Item A2) The method according to item A1, wherein the measurement of the inhibitory activity is performed using a cell in which both gene of sphingomyelin synthase SMS1 and SMS2 is knocked out.
(Item A3) The method according to item A2, wherein when the cell is given cyclodextrin and the subject substance causes delay in cell growth or cause cell death, the subject substance is determined to have said at least one function.
(Item A4) The method according to item A3, wherein the cyclodextrin is methyl beta cyclodextrin.
(Item A5) The method according to any one of items A2 to A4, wherein the cell is immortalized murine fibroblast.
(Item A6) The method according to any one of items A2 to A5, wherein the gene introduction is performed with a retrovirus.
(Item A7) The method according to any one of items A2 to A6, wherein said at least one sphingomyelin synthase is SMS1 or SMS2, or both SMS1 and SMS2.
(Item A8) The method according to any one of items A2 to A7, wherein said at least one sphingomyelin synthase is SMS2.
(Item A9) A kit for screening for a drug having at least one function selected from the group consisting of an anti-obesity drug, a drug for decreasing visceral fat, a drug for treating fatty liver and a drug for increasing adiponectin expression, comprising a cell in which both gene of sphingomyelin synthase SMS1 and SMS2 has been knocked out wherein at least one sphingomyelin synthase is genetically introduced thereinto.
(Item A10) The kit according to item A9, further comprising cyclodextrin.
(Item A11) The kit according to item A10, wherein the cyclodextrin is methyl beta cyclodextrin.
(Item A12) The kit according to item A9 to A11 wherein the cell is an immortalized murine fibroblast.
(Item A13) The kit according to any one of items A9 to A12, wherein the gene introduction is performed with a retrovirus.
(Item A14) The kit according to any one of items A9 to A13 wherein said at least one sphingomyelin synthase is SMS1 or SMS2, or both SMS1 and SMS2.
(Item A15) The kit according to any one of items A9 to A14 wherein said at least one sphingomyelin synthase is SMS2.
(Item A16) A cell in which sphingomyelin synthase, SMS1 and SMS2 are knocked out, wherein at least one of sphingomyeline synthase is genetically introduced thereinto.
(Item A17) The cell according to item A16, wherein the cell is an immortalized murine fibroblast.
(Item A18) The cell according to item A16 or A17 wherein the gene introduction is performed with a retrovirus.
(Item A19) The cell according to any one of items A16 to A18, wherein said at least one sphingomyelin synthase is SMS1 or SMS2, or both SMS1 and SMS2.
(Item A20) The cell according to any one of items A16 to A19 wherein said at least one sphingomyelin synthase is SMS2.

Relating to the present invention, it has been known that a metabolic syndrome result from complex factors. Therefore, it is likely that an occurrence of a medicament having a new mechanism of action can supplement the weakness of conventional drugs.

The present inventors have developed a method for ameliorating a metabolic syndrome by controlling sphingolipid metabolism, thereby indirectly affecting neutral lipid synthesis/metabolism, which has been not known to date, as well as a method for screening a inhibitor targeting the same.

The present inventors propose a method for ameliorating a metabolic syndrome by controlling sphingomyelin synthetases such as SMS2, a synthetase of sphingomyelin that is one of sphingolipids, to affect the synthesized amount of triacylglycerol. SMS produces equimolar amount of diacylglycerol in synthesizing sphingomyelin. From the experiments exemplified herein, it is demonstrated that the diacylglycerol is likely to be a material for the synthesis of triglycerol, a representative neutral lipid. Specifically, as an illustrative example, increase in body weight of SMS2 gene-deficient mice fed by a high fat diet was significantly decreased compared to a control group and closer to weight of mice fed by normal diet.

That is, it can be expected that by controlling the activity of SMS, the synthesized amount of triacylglycerol is controlled and consequently a metabolic syndrome are ameliorated. Further, gene deficient mouse of SMS2 developed by the present inventors, and cell lines isolated/produced therefrom may be used to screen for inhibitor of SMS2 having ameliorated effects on metabolic syndrome.

### [Advantageous Effects of Invention]

Conventionally, there have not been drugs for ameliorating a metabolic syndrome that are via controlling the synthesis of sphingolipids. Therefore, the present invention enables it possible to make a medicament having a new mechanism of action.

### [Brief Description of Drawings]

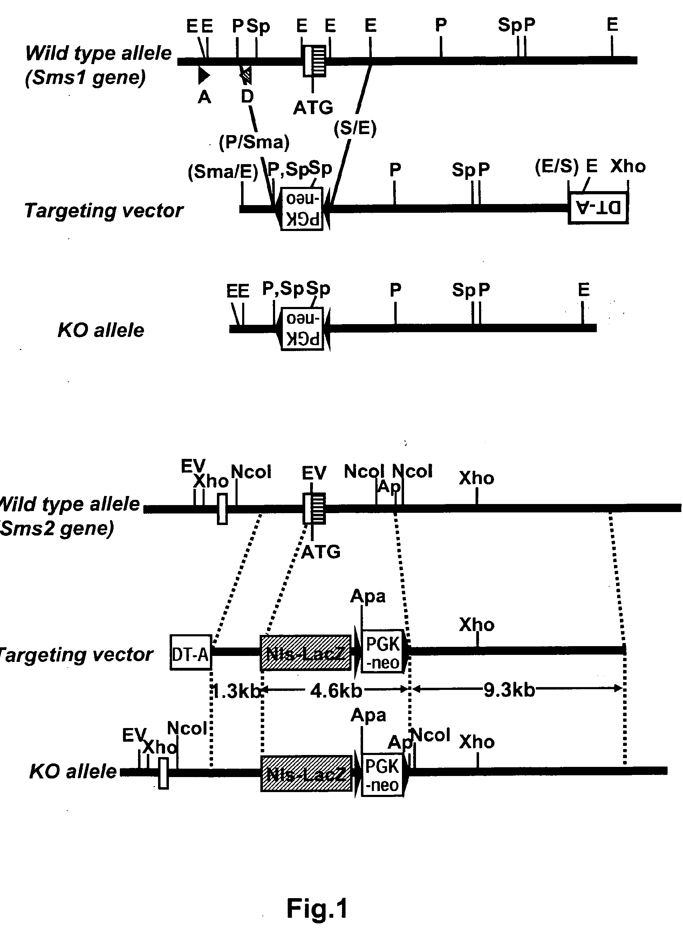

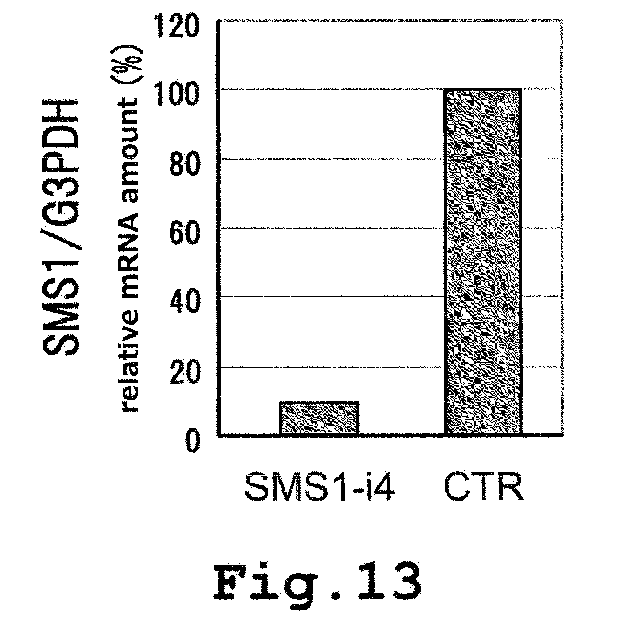

[Fig. 1] Figure 1 shows a summary of targeting vectors used in the production of SMSs knockout (KO) mice in Example 1. Top shows a summary of a targeting vector used in knocking out SMS1 gene, while bottom shows a summary of a targeting vector used in knocking out SMS2 gene. In the Figure, E refers to restriction enzyme cutting sites of EcoRI, P refers to those of PstI, S refers to those of SalI, Sp refers to those of SpeI, Sma refers to those of SmaI and Xho refers to those of XhoI.

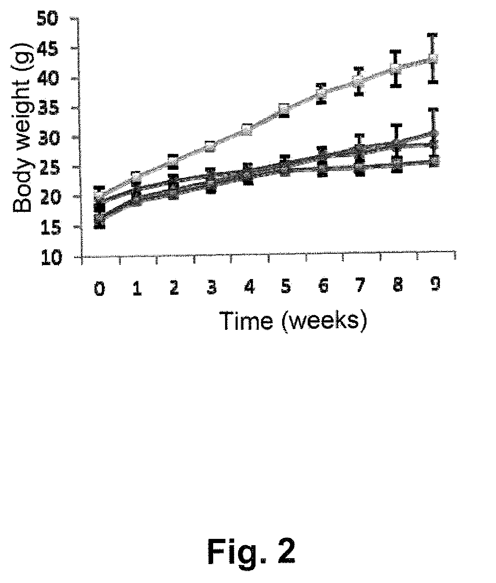

[Fig. 2] Figure 2 shows the result in Example 1 wherein the effect of 60% fat diet administration on body weight was investigated. The change of body weight (g) for 9 weeks is shown wherein high fat diet (HFD; 60% fat diet) or control normal diet (ND) were administered to wild-type (WT) and SMS2-KO mice. The wild-type mice to which high fat diet was administered (WT/HFD) increased their body weight, while in the SMS2-KO mice to which high fat diet was administered (SMS2 KO/HFD), increase in their body weight did not differ from that in wild-type mice to which normal diet was administered (WT/ND) and SMS2-KO mice to which normal diet was administered (SMS2 KO/ND) and increase in their body weight was significantly suppressed. Triangle shows WT/HFD; diamond, WT/ND; circle, SMS2 KO/HFD; square, SMS2 KO/ND.

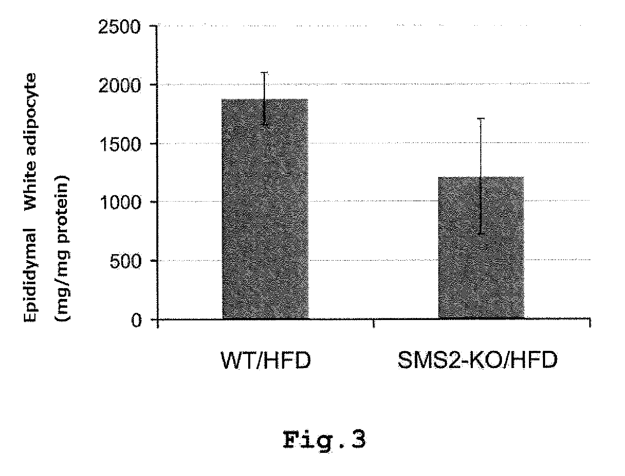

[Fig. 3] Figure 3 shows the result in Example 1 wherein the effect of 60% fat diet administration on white fat cell mass (WAT) was investigated. The comparison of weight (mg) of fat attached to epididymis is shown between wild-type (WT) and SMS2-KO mice to which high fat diet (HFD; 60% fat diet) were administered for 12 weeks. Compared to WT/HFD group (left), white fat cell mass (WAT) appeared to tend to decrease in SMS2 KO/HFD group (right), which was not a significant difference. Although not shown in the Figure, a similar experiment wherein a control normal diet (ND) was fed in parallel was also carried out. In the latter experiments, white fat cell mass (WAT) was decreased in SMS2 KO/HFD group compared to WT/HFD group, on the other hand, any significant difference was not found between the SMS2 KO/HFD group and the SMS2 KO/ND group.

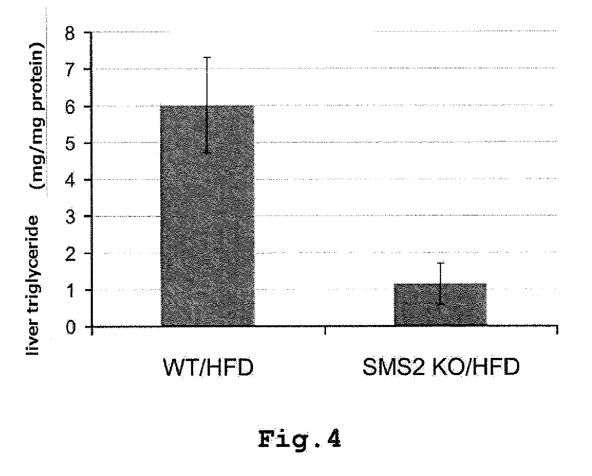

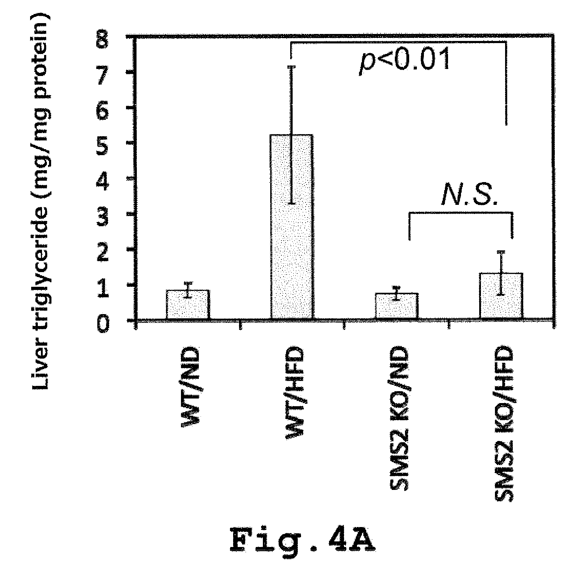

[Fig. 4] Figure 4 shows the result in Example 1 wherein the effect of 60% fat diet administration on liver triglyceride amount was investigated. The comparison of triglyceride amount in liver homogenate (mg/mg protein) is shown between wild-type (WT) and SMS2-KO mice to which high fat diet (HFD; 60% fat diet) were administered for 12 weeks. Compared to the WT/HFD group (left), the triglyceride amount was significantly decreased in the SMS2 KO/HFD group (right).
[Fig. 4A] Figure 4A shows the result in Example 1 wherein the effect of 60% fat diet administration on liver triglyceride amount was investigated. The comparison of triglyceride amount in liver homogenate (mg/mg protein) is shown between wild-type (WT) and SMS2-KO mice to which high fat diet (HFD; 60% fat diet) or control normal diet (ND) were administered for 12 weeks. Compared to WT/HFD group (left), the triglyceride amount was significantly decreased in SMS2 KO/HFD group (right). On the other hand, any significant difference was found between SMS2 KO/HFD group and SMS2 KO/ND group.

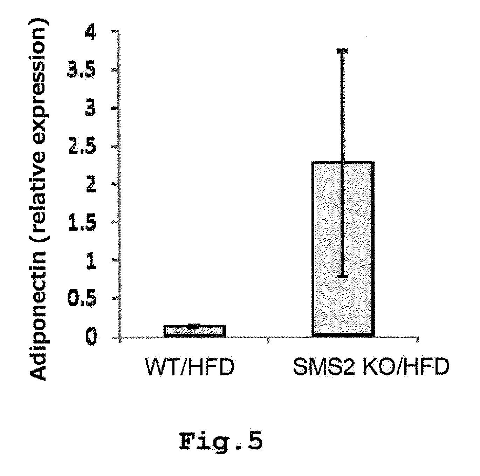

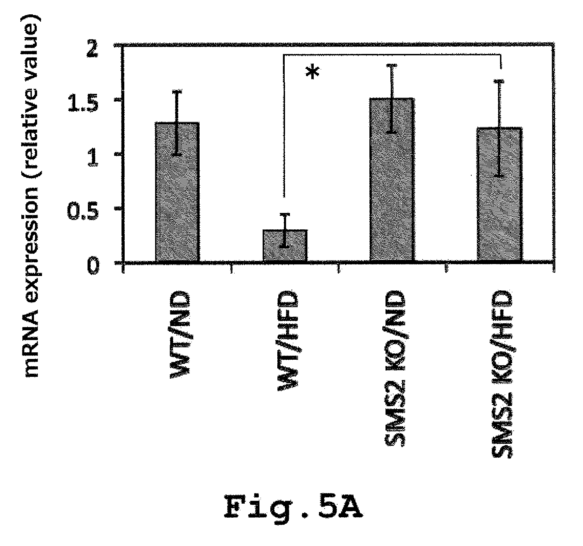

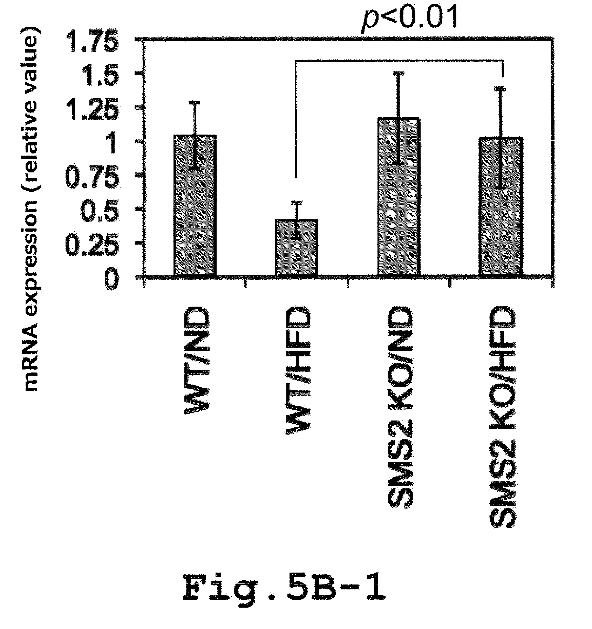

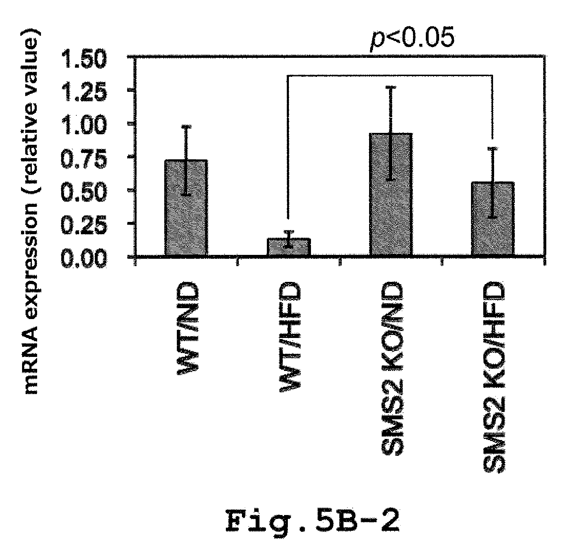

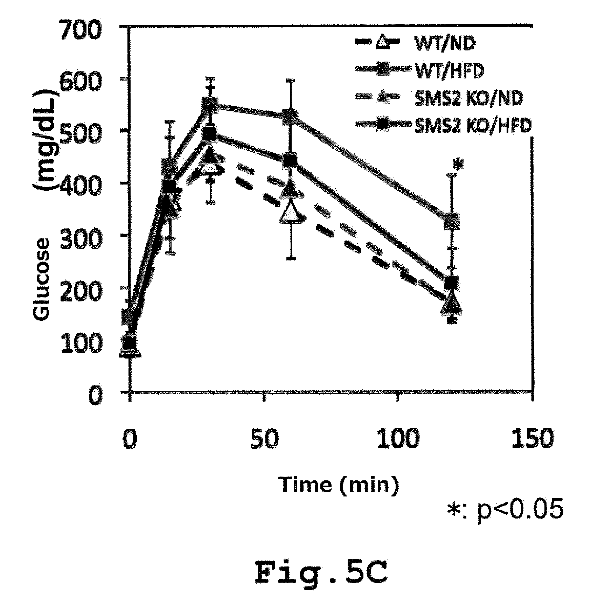

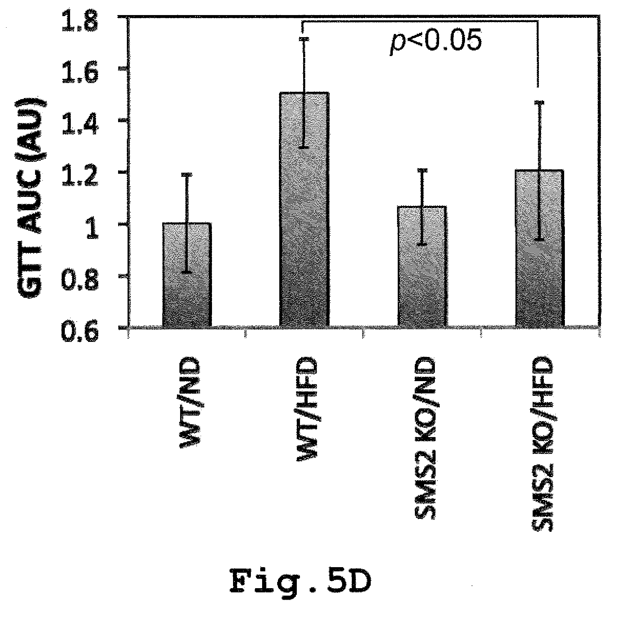

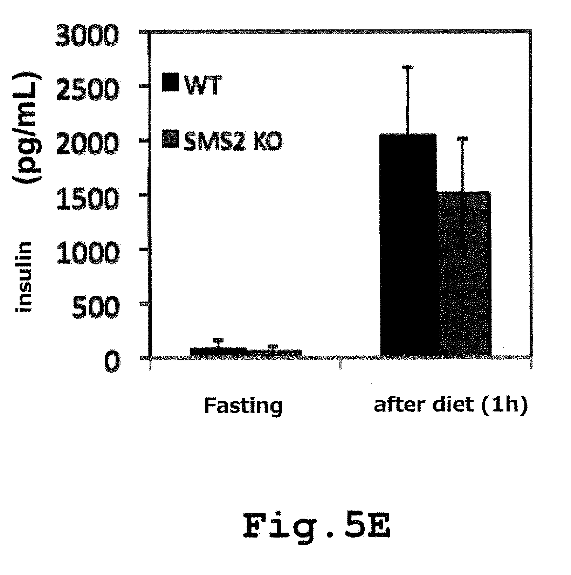

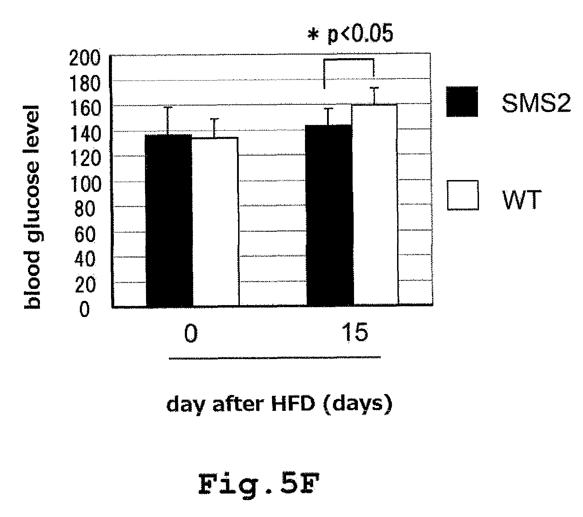

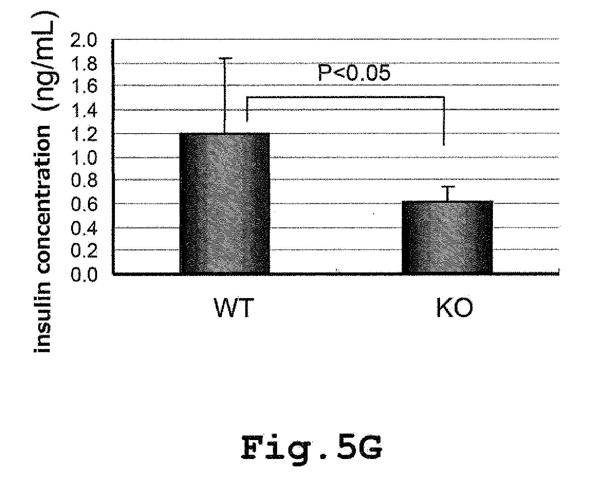

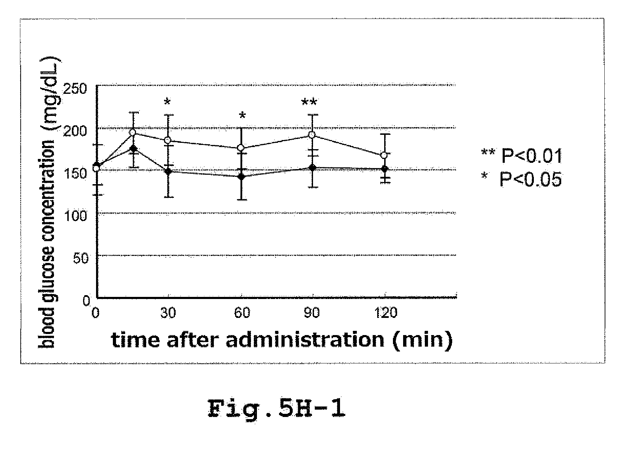

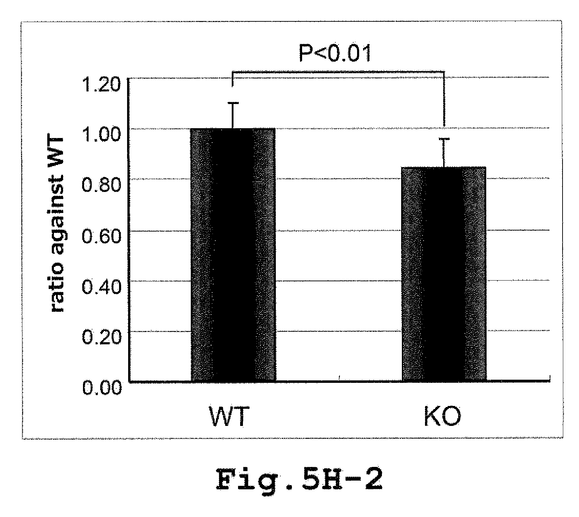

[Fig. 5] Figure 5 shows the result in Example 1 wherein the effect of 60% fat diet administration on adiponectin amount was investigated. The comparison of relative expression amount of adiponectin mRNA in adipose tissue is shown between wild-type (WT) and SMS2-KO mice to which high fat diet (HFD; 60% fat diet) were administered for 12 weeks. The expression of adiponectin was suppressed in WT/HFD group (left), while a relatively high expression amount was maintained in SMS2 KO/HFD group (right).
[Fig. 5A] Figure 5A shows the result in Example 1 wherein the effect of 60% fat diet administration on adiponectin amount was investigated. The comparison of relative expression amount of adiponectin mRNA in adipose tissue is shown between wild-type (WT) and SMS2-KO mice to which high fat diet (HFD; 60% fat diet) or control normal diet (ND) were administered for 12 weeks. In Figure 5A, in the order from left, shown are a group wherein wild-types were fed by normal diet (WT/ND), a group wherein wild-types were fed by high fat diet (WT/HFD), a group wherein SMS2-KO mice were fed by normal diet (SMS2 KO/ND), and a group wherein SMS2-KO mice were fed by high fat diet (SMS2 KO/HFD). Y axis shows relative amount of mRNA. In the WT group, by feeding high fat diet, the expression of adiponectin was suppressed, while in the SMS2 KO group, even when high fat diet was fed, a relatively high expression amount was maintained.
[Fig. 5B-1] Figure 5B-1 shows the result in Example 1 wherein the effect of 60% fat diet administration on an insulin receptor in adipose tissues was investigated. The comparison of relative expression amount of insulin receptor is shown between wild-type (WT) and SMS2-KO mice to which high fat diet (HFD; 60% fat diet) or control normal diet (ND) were administered. In the order from left, shown are a group wherein wild-types were fed by normal diet (WT/ND), a group wherein wild-types were fed by high fat diet (WT/HFD), a group wherein SMS2-KO mice were fed by normal diet (SMS2 KO/ND), and a group wherein SMS2-KO mice were fed by high fat diet (SMS2 KO/HFD). Y axis shows relative amount of mRNA. In the wild-type mice, when high fat diet (HFD; 60% fat diet) was fed, the expression amount of insulin receptor decreased. In SMS2-KO mice, decrease in the expression of insulin receptor did not occur by high fat diet and it was an almost similar expression amount to that of a case which control normal diet was fed. In addition, the expression amount of insulin receptor a case which high fat diet was administered to SMS2-KO mice was significantly higher compared to the case which high fat diet was administered to wild-type. From the above results, it was suggested that in adipose tissues of SMS2-KO mice, decrease in the expression of insulin receptor by high fat diet as observed in wild-type mice did not occur, and thus the SMS2-KO mice did not become insulin resistance.
[Fig. 5B-2] Figure 5B-2 shows the result in Example 1 wherein the effect of 60% fat diet administration on Glut4 in adipose tissues was investigated. The comparison of relative expression amount of Glut4 is shown between wild-type (WT) and SMS2-KO mice to which high fat diet (HFD; 60% fat diet) or control normal diet (ND) were administered. In the order from left, shown are a group wherein wild-types were fed by normal diet (WT/ND), a group wherein wild-types were fed by high fat diet (WT/HFD), a group wherein SMS2-KO mice were fed by normal diet (SMS2 KO/ND), and a group wherein SMS2-KO mice were fed by high fat diet (SMS2 KO/HFD). Y axis shows relative amount of mRNA. In the wild-type mice, when high fat diet (HFD; 60% fat diet) was fed, the expression amount of Glut4 decreased, while in SMS2-KO mice, the degree of decrease in the expression of Glut4 by high fat diet was suppressed and it was an almost similar expression amount to that of a case which control normal diet was fed. In addition, the expression amount of Glut4 when high fat diet was administered to SMS2-KO mice was significantly higher compared to when high fat diet was administered to wild-type. From the above results, it was suggested that in adipose tissues of SMS2-KO mice, decrease in the expression of Glut4 by high fat diet as observed in wild-type mice did not occur, and thus the SMS2-KO mice did not become insulin resistance.
[Fig. 5C] Figure 5C shows the result in Example 1 wherein the effect of 60% fat diet administration on blood glucose level was investigated. Wild-type (WT) and SMS2-KO mice were administered high fat diet (HFD; 60% fat diet) or control normal diet (ND) for 9 weeks, then fasted for 24 hours, then intraperitoneally administered 2g/kg glucose. Blood glucose levels were measured immediately before administration, 15 minutes after administration, at 30 minutes after administration, at 60 minutes after administration, and at 120 minutes after administration. Immediately before administration, no significant difference existed among the 4 groups, while at 120 minutes after administration, blood glucose level was significantly decreased in SMS2 KO/HFD group compared to in WT/HFD group. White triangle shows WT/ND, thin square shows WT/HFD, triangle that is filled in shows SMS2 KO/ND, and thick square shows SMS2 KO/HFD. Y axis shows blood glucose concentration (mg/dL), and X axis shows time (minute).
[Fig. 5D] Figure 5D represents the area under curve of Figure 5C (AUC) in bar graph. In the order from left, shown are a group wherein wild-types were fed by normal diet (WT/ND), a group wherein wild-types were fed by high fat diet (WT/HFD), a group wherein SMS2-KO mice were fed by normal diet (SMS2 KO/ND), and a group wherein SMS2-KO mice were fed by high fat diet (SMS2 KO/HFD). A significant difference was recognized between WT/HFD and SMS2 KO/HFD (p<0.05). Y axis shows relative values of GTT AUC (area=AU, unit is m² (area unit)).
[Fig. 5E]
   Figure 5E shows the result in Example 1 wherein the effect of normal diet (ND) on blood insulin amount was investigated. Wild-type (WT) and SMS2-KO mice were administered normal diet for 4 weeks, then fasted for 24 hours, then administered normal diet. Blood insulin concentration was measured at 24 hours fasting and 1 hour after normal diet administration. Left shows at fasting and right shows 1 hour after feeding. In each pair, left (thick column) shows wild-type (WT) and right (thin column) shows SMS2 KO. Y axis shows blood insulin concentration (pg/ml). Between wild-type (WT) and SMS2-KO mice, no significant difference in blood insulin concentration was observed at 24 hours fasting and 1 hour after normal diet administration. From the experiments, it can be said that even if SMS2 is knocked out, the amount of insulin and insulin secretion response itself to feeding are not affected. Therefore, any concern such as decrease in body weight and decrease in blood glucose level which is expected to result from abnormal secretion of insulin due to abnormality of beta cell of pancreas could be solved.
[Fig. 5F] Figure 5F shows the result in Example 1 wherein the effect of high fat diet on blood glucose level was investigated. 4 weeks old wild-type (WT) and SMS2-KO mice were administered high fat diet (HFD; 60% fat diet) for 2 weeks. Blood glucose level was measured at the time of 4 hours fasting before high fat diet administration and at the time of 4 hours fasting after 2 weeks of high fat diet administration. Left group shows Day 0 and right group shows Day 15. Black bars show SMS2-KO mice and white bars show wild-type (WT). Y axis shows blood glucose level (mg/dl). Although no significant difference in blood glucose level was observed before administering high fat diet between wild-type mice and SMS2-KO mice, blood glucose level in wild-type mice was significantly higher compared to in SMS2-KO mice after administering high fat diet for 2 weeks (p<0.05). From the results, it is meant that in SMS2-KO mice, blood glucose level was decreased with a significant difference. Combining with the results of Figure 5E, it can be said that if SMS2 is knocked out, diabetes with insulin resistance does not occur.
[Fig. 5G] Figure 5G shows the result in Example 1 wherein the effect of a high fat diet administration on blood insulin amount was investigated. Wild-type (WT) and SMS2-KO mice were administered high fat diet (HFD; 60% fat diet) for 2 weeks, and then fasted for 4 hours, and then blood insulin amount was measured. Left shows wild-type (WT) and right shows SMS2-KO mice (KO). Y axis shows blood insulin concentration (ng/ml). Consequently, in SMS2-KO mice, blood insulin amount was decreased with a significant difference compared to that in wild-type mice.
[Fig. 5H-1] Figure 5H-1 shows the result in Example 1 wherein the blood glucose concentration in response to insulin was investigated. 4 weeks old wild-type (WT) and SMS2-KO mice were administered high fat diet (HFD; 60% fat diet) for 6 weeks, at the time 6 weeks had elapsed, insulin were intraperitoneally administered in 0.5U/kg. The results of blood glucose concentrations that were measured before administration and at 15, 30, 60, 90 and 120 minutes after administration are shown. In particular, since blood glucose concentration in SMS2-KO mice was significantly decreased compared to in wild-type mice at 30, 60 and 90 minutes after administration, it became apparent that SMS2-KO mice were more sensitive to insulin. White circle shows wild-type (WT) and black circle shows SMS2-KO mice. * and ** both show significance (p<0.05 and p<0.01, respectively). Y axis shows blood glucose concentration (mg/dL) and X axis shows elapsed time after administration (minute).
[Fig. 5H-2] Figure 5H-2 shows a ratio of area under curve (AUC) in Figure 5H-1 to wild-type (WT) (WT=1) in a bar graph. Since it was significantly decreased by about 20% in SMS2-KO mice compared to in wild-type mice, it became apparent that SMS2-KO mice are more sensitive to insulin. From the above results of Figures 5F, G, H-1 and H-2, it can be said that if SMS2 is knocked out, it is resistant to diabetes with insulin resistance compared to WT.

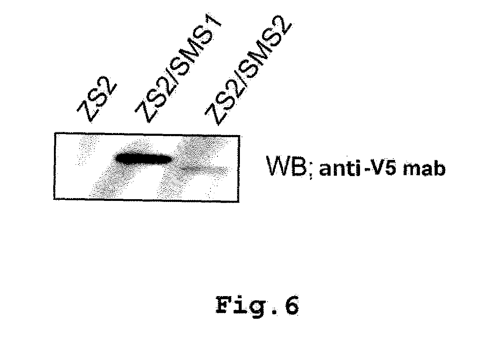

[Fig. 6] Figure 6 shows result of Western blotting wherein anti-V5 monoclonal antibody was used on ZS2 cell with SMS1 and SMS2 defects, SMS1-overexpresing cell (ZS2/SMS1) and SMS2-overexperssing cell (ZS2/SMS2) which were made in Example 2. In ZS2 cell, no band was detected, while in ZS2/SMS1 and ZS2/SMS2, bands of SMS1 and SMS2 which can be detected with anti-V5 mab were respectively confirmed.

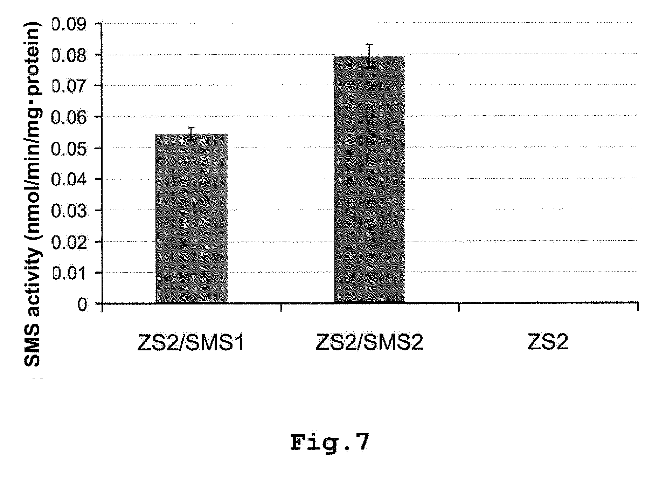

[Fig. 7] Figure 7 shows the result of investigation in Example 3 on SMS activity of ZS2 cell, ZS2/SMS1 cell and ZS2/SMS2 cell. ZS2/SMS1 cell (left) and ZS2/SMS2 cell (middle) had an SMS activity, while ZS2 cell (right) did not have any SMS activity. Vertical axis of the graph shows SMS activity (Unit: nmol/minute/mg protein).

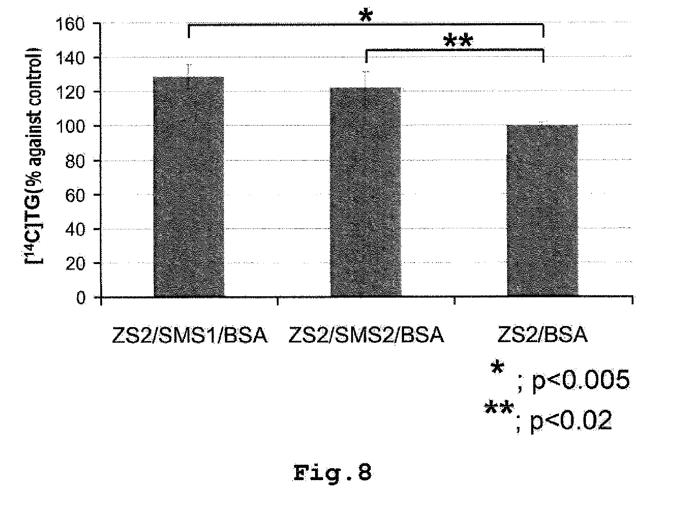

[Fig. 8] Figure 8 shows the result of investigation in Example 3 on TG synthesis by ZS2 cell, ZS2/SMS1 cell and ZS2/SMS2 cell. There was a significant difference between the TG amount in ZS2/SMS1 cell (left) and ZS2/SMS2 cell (middle), and the TG mount of ZS2 cell (right) (p<0.005 and p<0.02, respectively). Vertical axis of the graph shows percentage (%) of [¹⁴C]TG to control (ZS2 cell).

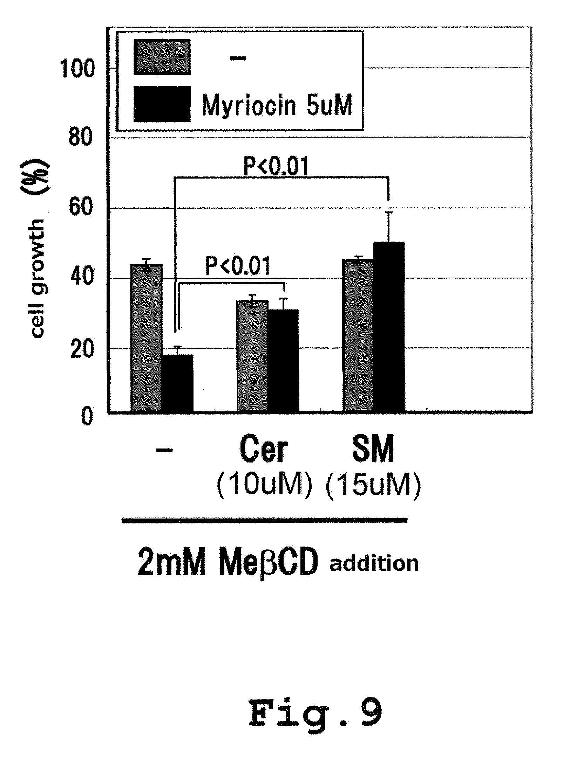

[Fig. 9] Figure 9 shows results of investigation where we studied changes of Me beta CD sensitivity using Myriocin, an inhibitor of synthesis of sphingolipid of ZS/SMS2 cells produced in Example 2 (n=3). Three groups are shown from the left side amongst which in one group, left shows one to which nothing has been added, and right shows one to which 5uM Myriocin has been added. The respective groups show, from the left hand, one to which 2mM Me beta CD only has been added, one to which ceramide (Cer) and 2mM Me beta CD have been added, and one to which sphingomyelin (SM) and 2mM Me beta CD have been added. Although significant difference was found in survival rate when Myriocin is added to the cells (two left-hand ones), no significant difference was found in survival rate by Myriocin when ceramide (Cer) or sphingomyelin (SM) is added to the medium. Therefore, it has been confirmed that the reduction of sphingomyelin by Myriocin increased sensitivity to Me beta CD sensitivity. Y axis represents a numerical value when the average value is deemed to be 100% amongst the cases in the presence and absence of Myriocin, when Me beta CD is not placed therein. There was not significant difference between the presence and absence of Myriocin, and thus the average of these has been made to be 100 %. The results are shown in Figure 9.

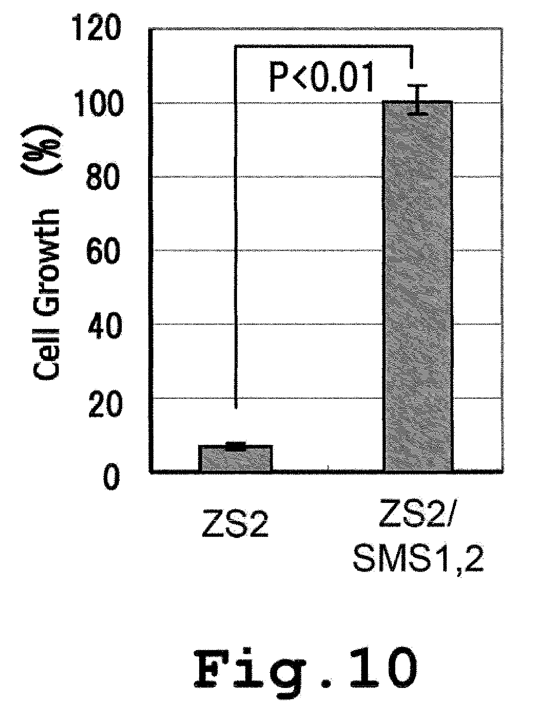

[Fig. 10] Figure 10 shows results of investigation where we studied sensitivity against Me beta CD of ZS2 and ZS2/SMS1,2 cells produced in Example 2. The left hand panel shows ZS2 cells, and the right hand panel shows ZS2/SMS1,2 cells. Y axis shows cell growth (%) (ZS2/SMS1,2 cells =100%). ZS2 cells were significantly more sensitive against ME beta CD in comparison to ZS2/SMS1,2 cells.

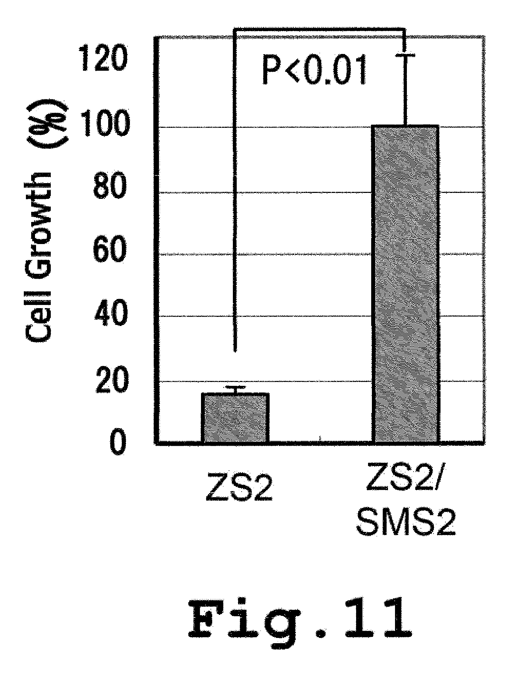

[Fig. 11] Figure 11 shows results of investigation where we studied sensitivity against Me beta CD of ZS2 and ZS2/SMS2 cells produced in Example 2. The left hand panel shows ZS2 cells, and the right hand panel shows ZS2/SMS2 cells. Y axis shows cell growth (%) (ZS2/SMS2 cells =100%). ZS2 cells were significantly more sensitive against ME beta CD in comparison to ZS2/SMS2 cells.

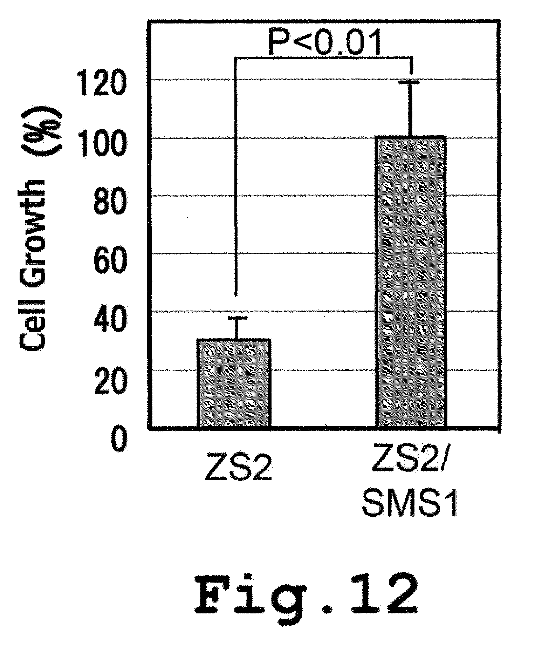

[Fig. 12] Figure 12 shows results of investigation where we studied sensitivity against Me beta CD of ZS2 and ZS2/SMS1 cells produced in Example 2. The left hand panel shows ZS2 cells, and the right hand panel shows ZS2/SMS1 cells. Y axis shows cell growth (%) (ZS2/SMS1 cells =100%). ZS2 cells were significantly more sensitive against ME beta CD in comparison to ZS2/SMS1 cells.
{Fig. 13] Figure 13 represents the results of investigation where we studied whether SMS1-i4, a siRNA against SMS1, inhibits the expression of SMS1 in Hepa1c1c7 cells (n=1). Y axis shows relative mRNA amount (%) of SMS1/G3PDH (CTR=100%). Cells to which SMS1-i4 was administered, has the expression of SMS1 more inhibited than those to which control siRNA was administered. Additionally, regarding the specificity in the mRNA level, it has been confirmed that the expression of SMS2 is not inhibited, and thus experimental data confirm the specificity against SMS1.

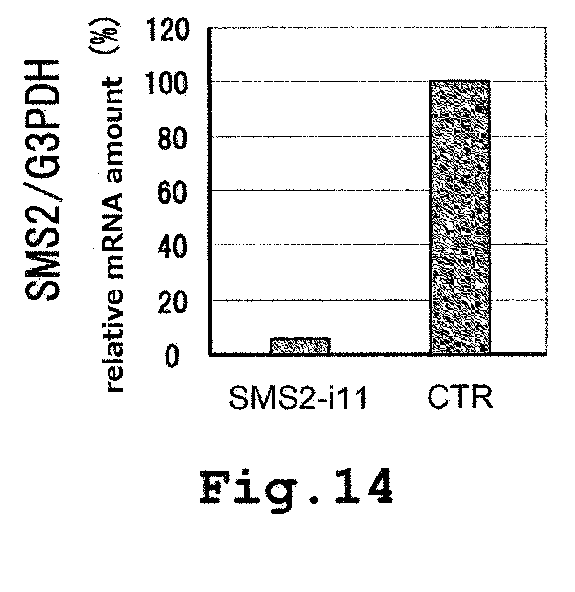

[Fig. 14] Figure 14 represents the results of investigation where we studied whether SMS2-i11, a siRNA against SMS2, inhibits the expression of SMS2 in Hepa1c1c7 cells (n=1). Y axis shows relative mRNA amount (%) of SMS2/G3PDH (CTR=100%). Cells to which SMS2-i11 was administered, has the expression of SMS2 more inhibited than those to which control siRNA was administered. Additionally, regarding the specificity in the mRNA level, it has been confirmed that the expression of SMS1 is not inhibited, and thus experimental data confirm the specificity against SMS2.

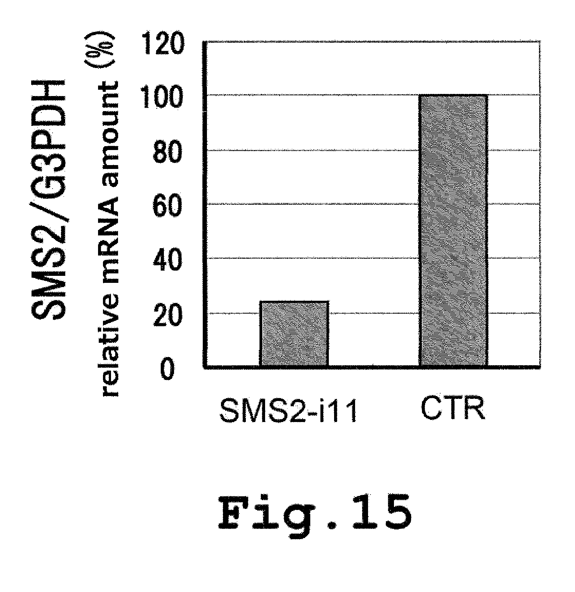

[Fig. 15] Figure 15 represents the results of investigation where we studied whether SMS2-i11, a siRNA against SMS2, inhibits the expression of SMS2 in ZS2/SMS2 cells produced in Example 2 (n=1). Y axis shows relative mRNA amount (%) of SMS2/G3PDH (CTR=100%). Cells to which SMS2-i11 was administered, has the expression of SMS2 more inhibited than those to which control siRNA was administered.

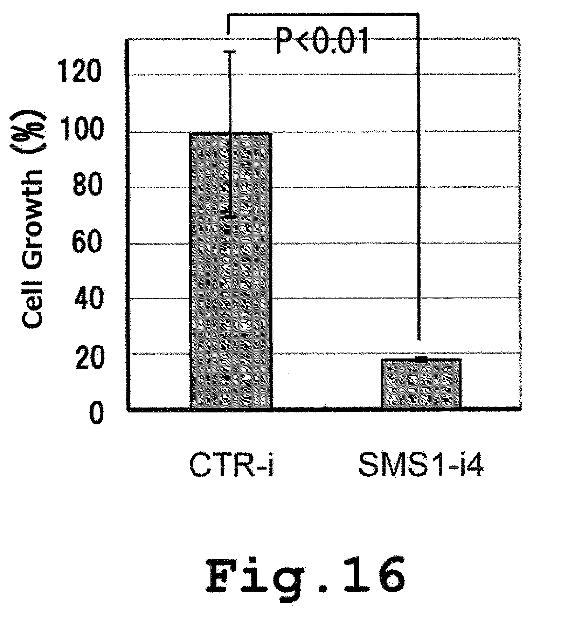

[Fig. 16] Figure 16 represents the results of investigation where we studied whether SMS1-i4, a siRNA against SMS1, changes sensitivity against Me beta CD by administration thereof to ZS2/SMS1 cells produced in Example 2. Left hand panel shows control siRNA (CTR-i), whereas right hand panel shows SMS1-i4. Y axis shows cell growth (%) (CTR-i=100%). Cells to which SMS1-i4 was administered were significantly (p<0.01) more sensitive than cell to which control siRNA was administered.

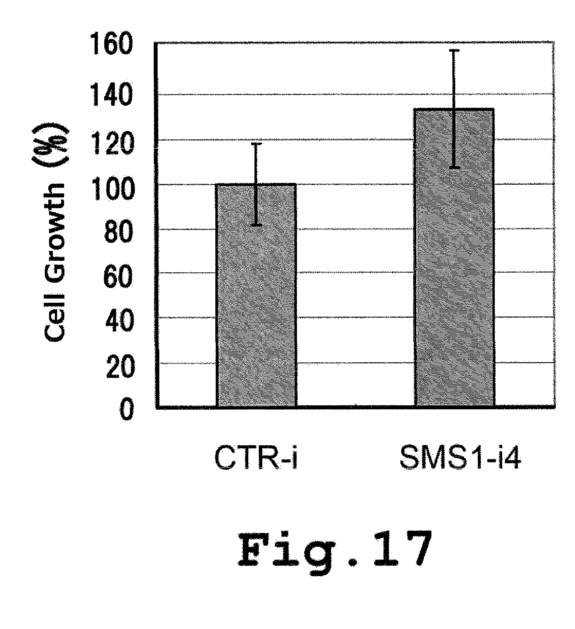

[Fig. 17] Figure 17 represents the results of investigation where we studied whether SMS1-i4, a siRNA against SMS1, changes sensitivity against Me beta CD by administration thereof to ZS2/SMS2 cells produced in Example 2. Left hand panel shows control siRNA (CTR-i), whereas right hand panel shows SMS1-i4. Y axis shows cell growth (%) (CTR-i=100%). There was no significant change in sensitivity to Me beta CD between cells to which SMS1-i4 was administered and cells to which control siRNA was administered.

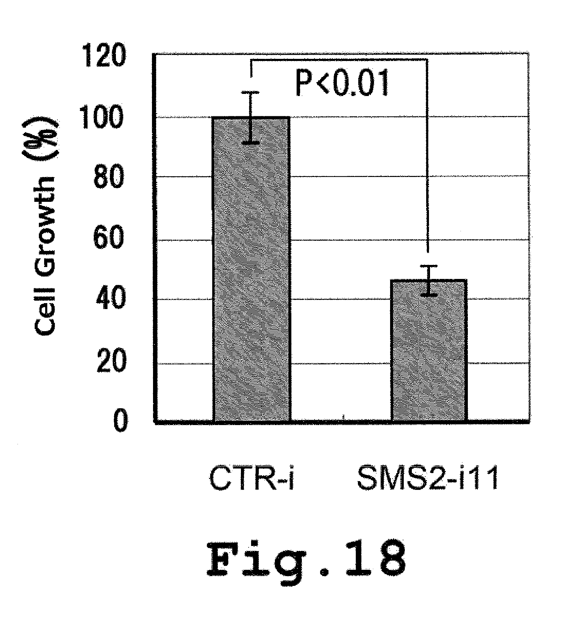

[Fig. 18] Figure 18 represents the results of investigation where we studied whether SMS2-i11, a siRNA against SMS2, changes sensitivity against Me beta CD by administration thereof to ZS2/SMS2 cells produced in Example 2. Left hand panel shows control siRNA (CTR-i), whereas right hand panel shows SMS2-i11. Y axis shows cell growth (%) (CTR-i=100%). Cells to which SMS2-i11 was administered were significantly (p<0.01) more sensitive than cell to which control siRNA was administered.

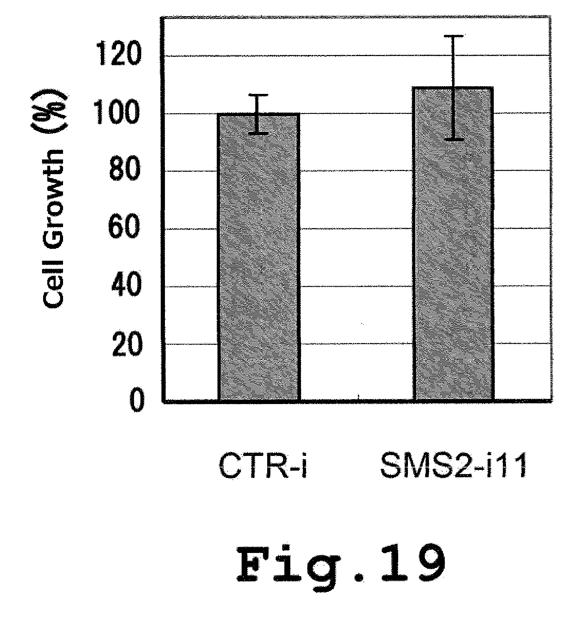

[Fig. 19] Figure 19 represents the results of investigation where we studied whether SMS2-i11, a siRNA against SMS2, changes sensitivity against Me beta CD by administration thereof to ZS2/SMS1 cells produced in Example 2. Left hand panel shows control siRNA (CTR-i), whereas right hand panel shows SMS2-i11. Y axis shows cell growth (%) (CTP-i=00%). There was no significant change in sensitivity to Me beta CD between cells to which SMS2-i11 was administered and cells to which control siRNA was administered.

### [Description of Embodiments]

Preferred embodiments of the present invention are explained below. Throughout the present specification, unless specifically referred to, an expression in a singular form is to be understood to encompass the concept of its plurality form. Therefore, unless specifically referred to, singular form articles (for example, "a", "an", "the" or the like in English, and corresponding articles and adjectives or the like in other languages) are to be understood to encompass the concept of their plurality form. Furthermore, terms used herein, unless specifically referred to, are to be understood to be used in the meaning usually used in the art. Therefore, unless defined otherwise, all technical terms and scientific terms herein have the same meaning as generally recognized by those skilled in the art to which the present invention belongs. If they contradict, the present specification (including the definition) governs.

### (Definition)

The definitions of terms specifically used herein are listed.

As used herein, "sphingomyelin synthetase" (also referred herein as "SMS") refers to an enzyme that synthesizes sphingomyelin (also referred herein as "SM") wherein it converts ceramide into sphingomyelin in the presence of phosphatidylcholine (also referred herein as "PC"), and which plays an important role in cell death and survival (see, NPL2 1 and 2). Here, phosphatidylcholine after conversion is converted into diacylglycerol. As sphingomyelin synthetases, typically, SMS1 and SMS2 are known, as well as homologs (see, NPLs 1 and 2). SMS1 is known to be expressed in the Golgi body and be involved in de novo synthesis of SM. On the other hand, SMS2 is expressed in the Golgi body and cell membrane, and its physiological function is not known in detail (see, NPL 3). In addition, GenBank Accession numbers of SMS1 are SM_147156 (human) and NM_144792 (mouse), and those of SMS2 are BC028705.1 (human), BC041369.2 (human) and NM_028943 (mouse).

As used herein, "a candidate substance" or "a subject substance" interchangeably used and both refer to any substance which is subject to a screening, and include but not limited to, for example, low molecular weight compounds (for example, including those synthesized by means of combinatorial chemistry and the like), organic compounds, peptides, polypeptides, proteins, carbohydrates, nucleic acids, lipids, and complexes thereof and the like.

As used herein, "inhibitory activity" refers, when referring to a substance, to that the substance at least reduces or loses an activity of a functional substance such as an enzyme (for example, sphingomyeline synthase). In the present invention, for example, the measurement of such an inhibitory activity may be performed by giving a cell death inducing agent such as cyclodextrin or the like to a cell of the present invention or the like and observing whether a candidate substance subject to the measurement causes delay of cell growth or cell death.

"Metabolic syndrome" or "metabolic disease" herein are used in the broadest meaning used in the art, and refer to states wherein hyperglycemia, dyslipidemia or hypertension are caused with a common factor of visceral fat obesity and are previously called as "lifestyle related disease", "adult diseases" or the like. All these terms are exchangeably used herein. Metabolic syndrome or metabolic disease herein specifically comprises at least one of diseases such as diabetes, obesity, visceral fat (dyslipidemia (excess of visceral fat)), fatty liver or states such as decreased expression of adiponectin. Furthermore, since arteriosclerosis is often developed after some years as a result of metabolic syndrome, it is understood that "metabolic syndrome" does not comprise arteriosclerosis in general but comprises the arteriosclerosis caused by metabolic syndrome (for example, diabetes, obesity or the like).

"Anti-diabetes" herein refers to a drug capable of treating (for example, suppressing, preventing or the like) diabetes. Diabetes is used in the meaning usually used in the art and refers to a state wherein a blood glucose level (glucose concentration in blood) is pathologically high, and diabetes is largely divided into type 1 diabetes which is caused by not secreting insulin from the pancreas and type 2 diabetes which is caused by decreased function of insulin. In the present specification, diabetes encompasses type I and type II and is not constricted to its type. In the present specification, "type I anti-diabetes" may be referred when specifically referring to type I and "type II anti-diabetes" may be referred when specifically referring to type II.

Here, insulin resistance syndrome is a major cause of diabetes (in particular, type II diabetes) and is a fundamental cause for lifestyle related diseases such as obesity and circulatory system disease. Insulin resistance means a state which a response to insulin of peripheral tissues is dull. As a result of a response to insulin being dull, since peripheral tissues cannot respond to insulin, they cannot uptake blood glucose and state in which blood glucose level is high is maintained. Since pancreas releases a large amount of insulin so as to decrease blood glucose, the concentration of insulin in blood becomes high.

Therefore, by ameliorating insulin resistance, blood glucose level decreases and thus it is useful for treating or preventing the above lifestyle related diseases. An index of amelioration of insulin resistance is that the concentration of insulin in blood is decreased when measured. In addition, increased concentration of adiponectin in blood is another index. In view of above, a drug for increasing adiponectin or a drug which decreases the concentration of insulin in blood while maintaining blood glucose level at a normal level becomes a drug for ameliorating insulin resistance and thus is also effective for treating diabetes or obesity, circulatory system diseases (arteriosclerosis, hypertension or the like) and metabolic syndrome resulting from insulin resistance.

"Anti-obesity drug" herein refers to a drug capable of treating (for example, suppressing, preventing or the like) obesity. Obesity state can include, for example, excess of body weight, excess of fat or the like. If BMI measured as a body mass index is 25 or more, it is deemed as obesity. When body fat percentage is used, 18% or more is a criterion. An effect of treating obesity can be judged by measuring body weight, measuring fat, measuring triglyceride accumulated in fat, or the like.

"Drug for decreasing body weight" herein refers to a drug which decreases body weight or suppresses feeding. "decrease in body weight" or "decreasing body weight" herein must be interpreted in the broadest manner to encompass a case of decreasing the current body weight as well as a case of suppressing increase in body weight and maintaining the current body weight. Decrease in body weight can be judged by measuring body weight, measuring fat, measuring triglyceride accumulated in fat, or measuring the amount of feeding.

"Drug for decreasing visceral fat" herein refers to a drug which decreases visceral fat or prevent increase in visceral fat. Visceral fat is used in the meaning usually used in the art and refers to fat accumulated around viscera among body fat. Decrease in visceral fat can be judged by measuring white fat cell mass, measuring with abdominal CT image, or the like.

"Drug for treating fatty liver" herein refers to a drug capable of treating (for example, suppressing, preventing) fatty liver. "Fatty liver" is used in the meaning usually used in the art and refers to a status in which a large amount of fat accumulates in the liver. As a cause for it, alcoholic, nutritive, diabetic, drug-induced causes or the like are known. Fatty liver is said to be partly developed to cirrhosis. In the sense, the present invention can be recognized to be a drug for preventing cirrhosis. An effect of treating fatty liver can be judged by measuring triglyceride (TG) in fat, measuring liver weight, grossly inspecting liver, or the like.

"Drug for increasing adiponectin expression" herein refers to a drug which increases the expression of adiponectin. Adiponectin is used in the meaning usually used in the art, is a secretory protein that is secreted from fat cell and is said to be negatively correlated with visceral fat amount, and said to be an index for so-called metabolic diseases. Increase in adiponectin expression can be judged, for example, by obtaining fat, extracting mRNA thereof and measuring the expression amount of adiponectin mRNA, by immunological measurement using an anti-adiponectin antibody, or the like.

"Expression" of a gene, polynucleotide, polypeptide or the like herein refers to that such gene or the like undergo in vivo a certain action to become another form. Preferably, it refers to that gene, polynucleotide or the like are transcribed and translated into a polypeptide form. It can be also one embodiment of the expression that they are transcribed so as to produce mRNA. More preferably, such forms of polypeptide can be one after post-translational processing.

Therefore, "decrease" in "expression" of gene, polynucleotide, polypeptide or the like herein refers to that the amount of expression is significantly decreased when the agent of the present invention is acted, compared to a case it is not acted. Preferably, decrease in expression encompasses decrease in the expression amount of a polypeptide. "Increase" in "expression" of gene, polynucleotide, polypeptide or the like herein refers to that the amount of expression is significantly increased when the agent of the present invention is acted, compared to a case it is not acted. Preferably, increase in expression encompasses increase in the expression amount of a polypeptide. "Induction" of "expression" of a gene herein refers to that a cell is acted by an agent so as to increase the expression amount of the gene. Therefore, induction of expression encompasses allowing the gene to be expressed when the expression of the gene is not found at all, and increasing the expression of the gene when the expression of the gene has been already found.

"Detection" or "quantification" of a gene expression (for example, mRNA expression, and polypeptide expression) herein can be achieved by using a suitable method, for example, including measurement of mRNA and immunological measuring methods. As molecular biological measuring methods, for example, Northern blot method, dot blot method or PCR method or the like are exemplified. As immunological measuring methods, for example, ELISA method using a microtiter plate, RIA method, fluorescent antibody method, Western blot method, immunohistological staining method or the like are exemplified. In addition, as a quantifying method, ELISA method or RIA method or the like are exemplified. It can be also carried out by a gene analyzing method using an array (for example, DNA array, and protein array). A DNA array is broadly in reviewed in (Shujunsha Co., Ltd., ed., Saibo Kogaku Bessatsu [Cell Engineering, Separate zvolume], "DNA Maikuroarei to Saishin PCR Ho [DNA Microarray and the Latest PCR Method]"). A protein array is described in detail Nat Genet. 2002 Dec; 32 Suppl: 526-32. In addition to the above, a method for analyzing gene expression include, but not limited to, RT-PCR, RACE method, SSCP method, immunoprecipitation, two-hybrid system, in vitro translation or the like. Additional such analyzing method is described, for example, in Genomu Kaiseki Jikken Ho [A Experimental Method for Genome Analysis], Nakamura Yusuke Rabo Manyuaru [Laboratory Manual by Yusuke Nakamura], Yusuke Nakamura ed., Yodosha Co., Ltd. (2002), the descriptions of which are all incorporated herein as references.

"Expression amount" herein refers to an amount in which a polypeptide or mRNA is expressed in a cell of interest or the like. Such expression amount includes expression amount of the polypeptide of the present invention at a protein level evaluated by any suitable methods using the antibody of the present invention including immunological measuring methods such as ELISA method, RIA method, fluorescent antibody method, Western blot method, immunohistological staining method or the like, or expression amount of the polypeptide of the present invention at an mRNA level evaluated by any suitable method including molecular biological measuring methods such as Northern blot method, dot blot method, PCR method or the like. "Change of expression amount" means increase or decrease in expression amount of a polypeptide of present invention at a protein level or mRNA level evaluated by any suitable method including the above immunological measuring methods or molecular biological measuring methods.

"Transgenic" herein refers to integration of a specific gene into an organism (or cell or the like), or an organism (for example, including animals (such as a mouse)) (or cell or the like) wherein such gene is integrated or defective or suppressed. Among transgenic organisms (or cell or the like), one wherein a gene is defective or suppressed is referred to as a knockout organism (or cell or the like). Therefore, "knockout", when referring to animal or cell or the like, means states wherein a native gene which is targeted does not function or is not expressed.

"Transgenesis" or "gene introduction" or "gene transfer" herein refers to introducing a gene of interest into a cell, tissue or animal, conceptually encompasses "transformation", "transduction" and "transfection" or the like, and can be realized by any technique known in the art. In addition, "transgenesis" also encompasses any of one wherein a site to be introduced is not limited and one via homologous recombination wherein a site to be introduced is limited. A method for transgenesis include, but not limited to, for example, methods using retrovirus, plasmid, vector or the like, or electroporation method, methods using particle gun (gene gun), calcium phosphate method. A cell used for transgenesis can be any cell, and use of an undifferentiated cell (for example, fibroblast or the like) is preferred.

As used herein, "screening" refers to a selection of a substance having a particular property of interest (such as a drug) from a number of candidates by a particular operation and/or evaluation processes. With respect to the present invention, it should be understood that a drug or a candidate therefor having a desired activity obtained by the screening are included in the scope of the present invention.

"Preventing" herein refers to, by any means, not allowing the occurrence of or at least delaying a disease, disorder or symptom which the present invention targets before occurrence of the disease, disorder or symptom, or not allowing the occurrence of a disorder even if any cause itself of the disease, disorder or symptom occurs.

"Therapy treating" herein refers to stopping the progression of a disease, disorder or symptom which the present invention targets, or completely or partly arresting or ameliorating a disease, disorder or symptom which the present invention targets.

"Treatment" herein broadly refers to somewhat affecting a disease, disorder or symptom or preventing a subject from being in such a disease, disorder or symptom, and can encompass therapy and prevention. In narrower sense, "treatment" refers to the above action after occurrence thereof compared to "prevention".

"Cell death inducing drug" refers to any drug capable of inducing cell death. In the present invention, any material can be used as long as it is a drug capable of inducing cell death by biding to cholesterol in cell membrane and destroying the cell membrane. In an SMS deficient cell, cyclodextrins can be used so as to induce cell death, but it is understood that the drug is not limited to them. "Cyclodextrin" as referred to herein is used in broader sense, refers to any cyclic oligosaccharide and derivatives thereof which several molecules of D-glucose are bound via alpha(1->4) glycoside bond, and refers to any one of substituted (for example, substituent includes, but not limited to, alkyl group, hydroxylalkyl group or the like) or unsubstituted cyclodextrins. Cyclodextrin which can be used as a cell death inducing drug includes, for example, methyl-alpha-cyclodextrin, methyl-beta-cyclodextrin, 2-hydroxylpropyl-beta-cyclodextrin, alpha-cyclodextrin, beta-cyclodextrin or the like (for reference, see, J.Biol.Chem. 270, 29, 17250-17256, 1995; J.Biol.Chem. 275, 44, 34028-34034; Japanese Laid-Open Publication No. 2007-332128 or the like).

"Reconstructed cell" herein refers to a cell produced by introducing a gene of interest into it, optionally after deleting a specific gene by knocking out it. Reconstructed cell can include cells wherein at least one sphingomyelin synthetase (for example, SMS1, SMS2 or both of SMS1 and SMS2) are transgenically introduced into a cell wherein the sphingomyelin synthetase SMS1 and SMS2 of the present invention are knocked out, or the like.

### (Description of Preferred Embodiments)

It should be understood that while description of preferred embodiments is described below, the embodiments are illustrative of the present invention and the scope of the present invention is not limited to such preferred embodiments. It should be also understood that those skilled in the art can readily carry out modification, alternation or the like within the scope of the present invention with reference to the preferred embodiments below.

### (Methods for discovering a drug)

In one aspect, the present invention provides a method for screening a substance effective for treating or preventing a metabolic syndrome. The present method comprises the step of measuring inhibitory activity of a subject substance against sphingomyelin synthase, wherein when the subject substance has an inhibitory activity of a sphingomyelin synthase, the subject substance is determined to be effective for treating or preventing the metabolic syndrome (for example, at least one function selected from the group consisting of an anti-diabetes, an anti-obesity drug, a drug for decreasing body weight, a drug for decreasing visceral fat, a drug for treating fatty liver and a drug for increasing adiponectin expression).

In the present method, the measurement of the inhibitory activity against sphingomyelin synthase is carried out by any method known in the art or described herein. For example, known methods may be isolating only an enzyme and assaying such an enzyme, however, this is not practical. As such, in the laboratory level, a method may be used wherein ceramide (substrate for SMS), which is labeled with fluorescence, is incorporated into a cell, and crude lipid is extracted from the cell, and the lipid is separated by thin layer chromatography (TLC) and SM labeled with fluorescence is measured. Alternatively, a reconstituted cell of the present invention such as a cell in which only one of sphingomyelin synthase SMS1 or SMS2 is expressed (for example, a cell in which either one gene of sphingomyelin synthase SMS1 or SMS2 is knocked out), or a cell in which either one gene of sphingomyelin synthase SMS1 or SMS2 is knocked out and has at least one sphingomyelin synthase gene introduced therein, or the like, may be used to confirm whether the cell is dead or the growth thereof is inhibited after giving a cell death inducing agent.

In the present invention, experiments using a knock-out animal in which sphingomyelin synthase, in particular SMS2 is knocked out, showed that such a sphingomyelin synthase is related to diabetes (such as type II diabetes mellitus, insulin-resistance syndrome, Impaired Glucose Tolerance), obesity (increase in body weight), increase in visceral fat, fatty liver, reduction in expression of adiponectin. Accordingly, it is understood that by inhibiting sphingomyelin synthase, in particular SMS2, amelioration, effects regarding treatment or prevention of metabolic syndrome are attained, such as in particular, amelioration, treatment or prevention of diabetes (such as type II diabetes mellitus, insulin-resistance syndrome, Impaired Glucose Tolerance), treatment or prevention of obesity, reduction, or prevention of increase in visceral fat, treatment or prevention of fatty liver, increase, or prevention or reduction in expression of adiponectin.

While not desiring to be bound by any theory, SMS produces equimolar diacylglyceride upon synthesis of sphingomyelin, and the present invention showed that diacylglycerol may be a starting material for triglycerol synthesis, which is a representative neutral lipid. Specifically, increase in body weight upon loading high fat feed was fed to mice with SMS2 gene defects, is significantly reduced than the control group, and is similar to those upon loading with normal feed. Accordingly, while not desiring to be bound by any theory, it is understood that by controlling the activity of SMS thereby controlling synthesis amount of triacylglycerol, metabolic syndrome such as diabetes (in particular type II diabetes mellitus, insulin-resistance syndrome, Impaired Glucose Tolerance), obesity, increase in visceral fat, fatty liver and the like may be consequently ameliorated. Metabolic syndrome is as defined herein above, while arterial sclerosis is a condition where a variety of symptoms by occlusion of blood vessels (for example, angina pectoris, myocardial infarct, cerebral infarction, and retinopathy and vascular occlusion due to diabetes and the like). In order to distinguish these in terms of diacylglycerol of SMS2, it is believed that knocking-out of SMS2 causes inhibition of triacylglycerol synthesis in liver by inhibiting production of diacylglycerol, thereby preventing onset of fatty liver, diabetes, obesity and the like. As a result of being metabolic syndrome, there are a number of cases developing arterial sclerosis after several years. Therefore, "metabolic syndrome" does not include arterial sclerosis in general. However, it is understood that metabolic syndrome includes arterial sclerosis caused by the metabolic syndrome (for example, diabetes, obesity and the like). Prior art such as those described in NPLs 4-6 were intended to inhibit arterial sclerosis by antiinflammatory action and thus did not focus on metabolic syndrome. On the other hand, the present invention is significantly distinct from the prior art in that inhibition of triacyl glycerol in liver by inhibition of production of diacylglycerol as an inhibitory mechanism of metabolic syndrome has now been found.
Moreover, animals such as SMS2 gene deficient mice and the like, cell lines isolated/produced therefrom may be used to screen inhibitor of SMS2 having amelioration effects of metabolic syndrome. When using cells, as exemplified in the Examples and as described hereinbelow, a cell in which either one of genes of sphingomyelin synthase SMS1 or SMS2 is knocked out, or a cell in which SMS2 gene of interest has been introduced into the knocked out cell, or a cell in which at least one sphingomyelin synthase is genetically introduced into a cell in which sphingomyelin synthases SMS1 and SMS2 have been knocked out, or the like can be used to perform the present invention.

SMS enzymes take a complex structure having Golgi apparatus or six-transmembrane in a cellular membrane. As such it is believed that it is difficult to perform an assay by isolating only the enzyme. In the present invention, a method is used wherein ceramide substrate for SMS which is fluorescently labeled is incorporated into a cell, and crude lipid is extracted from the cell, and the crude lipid is separated by means of thin chromatography (TLC) and the like, and quantification of fluorescent SM produced is performed.

In one embodiment, in the present invention, the measurement of inhibitory activity may be performed using a cell in which only one of sphingomyelin synthase SMS1 or SMS2 is expressed (such as a cell in which either one gene of sphingomyelin synthase SMS1 or SMS2 is knocked out), or a cell in which either one gene of sphingomyelin synthase SMS1 or SMS2 is knocked out and has at least one sphingomyelin synthase genetically introduced therein. In a conventional method as described above, in which fluorescently labeled ceramide (substrate for SMS) is incorporated into a cell, is convenient in a laboratory level, however, it is believed to be difficult to screen for a inhibitor in a large scale since operations such as lipid extraction and development with TLC and the like are carried out. Therefore, using such a cell line is advantageous in terms of operation and thus preferable. When using a cell as in the present embodiment, there is difference in growth capability, and thus it is possible to confirm the presence or absence of inhibition using a plate reader by addition a reagent for measuring growth, for example. As such, there is "high through-put" in comparison to the conventional art, and thus can be made in a large scale.

In one embodiment, the measurement of the inhibitory activity determines that when the cell is given a cell death inducing agent (for example, cyclodextrin such as methyl beta cyclodextrin) and causes delay in cell growth or cause cell death, the candidate substance is determined to have said at least one function. As used herein, cell death inducing agents available in the present invention may be any type as long as such an agent induces cell death by binding to cholesterol in a cell membrane thereby disrupting the cell membrane, and cyclodextrins may be used. Alpha cyclodextrin, methyl beta cyclodextrin and the like are used and assay system using the same are already developed in J. Biol. Chem. 270, 29, 17250-17256, 1995; J. Biol. Chem. 275, 44, 34028-34034; and Japanese Laid-Open Publication No. (A) 2007-332128, and methods described therein may be used to perform the present invention but are not limited thereto.

Cells used in the present invention may be any cells so long as such a cell expresses only either of sphingomyelin synthase SMS1 or SMS2. Starting cells from which such a cells may be produced, may be any cells as long as such a cell may be knocked out and gene introduction may be performed. For example, such a cell may be undifferentiated cells such as stem cells, fibroblasts and the like, or differentiated cells, and not particularly limited thereto. As an example, fibroblast (for example, mouse embryonic fibroblast (also referred to as "MEF") from fetus in which SMS1 and SMS2 are both knocked out. Fibroblasts may be prepared by referring to a reference, for example, Shin-baiyo saibo jikken ho (New methods for cell culture experiments) (Yodo-sha), 4th Section, Primary Culture (1) Fibroblast, pp. 66-70. Alternatively, mouse embryonic fibroblast (also referred to as "MEF") and the like taken from mouse fetus with only SMS1 or SMS2 defect may be used as a cell in which sphingomyelin synthase SMS1 or SMS2 only is expressed. Furthermore, such a knock out cell may be used to produce a cell in which one gene of either sphingomyelin synthase SMS1 or SMS2 is knocked out. It is understood that such a cell may be used to produce a cell for screening for a substance which is effective for treating or preventing metabolic syndrome.

In one embodiment, the cell used may be immortalized. Immortalization allows stable screening. As a method for immortalization, any methods known in the art may be used, for example, methods using SV40T as described in Proc. Nati. Acad. Sci. USA Vol.85, pp. 6450-5464,September 1988 and methods using HPV as described in Shin baiyo saibo jikken ho (New methods for cell culture experiments) (Yodo-sha), page 277, but are not limited thereto. For example, retrovirus placed in culture serum of 293T cells which stably holds plasmid pMFG-SV40Tst is infected to fetal MEF with double knock-out of both SMS1 and SMS2 defects to immortalize the MEF.

As a virus producing cell used in the present invention, any cells known in the art may be used. For example, NIH3T3 ecotropic producer cell ΨCRE-MFGtsT (SV40 T antigen expression virus producing cell; available from RIKEN(http://www2.brc.riken.jp/labfcell/detail.cgi?Cell r2o= RCB1119&type=1), a variety of virus cell producing cells available from American Type Culture Collection (ATCC) may be used.

As an example of one of immortalization, ΨCRE-MFGtsT cells release retrovirus expressing SV40tsT into a medium, and infects with mouse host cells. This retrovirus is used to introduce SV40tsT gene into MEF to achieve immortalization.

In the process of producing cells used in the present invention, when necessary, cloning of the immortalized cells are performed by using methods known in the art.

In the present invention, any method known in the art may be used to perform gene introduction, and for example, methods using retrovirus, plasmids, vectors, and the like, or alternatively electroporation methods, methods using particle gun (gene gun), calcium phosphate method and the like, but are not limited thereto. For example, as SMS1,2 expression vector, pQCXIP found in Retro-X^{™} Q Vector Set (Clontech) may be used and Retro-X^{™} Universal Packaging System (Clontech) may be used to produce/cause infection to achieve gene introduction.

In a cell used in the present invention, at least one of sphingomyelin synthases to be genetically introduced may be SMS1, SMS2, or both SMS1 and SMS2, or alternatively homologs thereof. In one embodiment, said at least one sphingomyeline synthase is SMS2. Additionally, as shown in the Examples, SMS2 has been demonstrated to be relevant to an anti-diabetes (such as drugs for type II diabetes mellitus, drugs ameliorating insulin-resistance (including insulin-resistance syndrome, Impaired Glucose Tolerance and the like), an anti-obesity drug, a drug for decreasing visceral fat, a drug for treating fatty liver and a drug for increasing adiponectin expression.

In an exemplary embodiment, an assay relating to cell survival and growth rate may be performed as described in NPL1. Specifically, 1 x 10⁶ cells of MEF cells are washed and resuspended into 1 ml of serum-free RPMI 1640 medium, and treated with an appropriate concentration of Me beta CD, and culture at 5 % CO₂, 37 degrees Celsius for five minutes. Two mls of normal medium are added and the cells are culture for additional 12 hours. For example, live cell number counting reagent including WST-8 which is reduced by intracellular dehydrogenase, and produces water soluble formazan (Nacalai Tesque) may be used to measure survival rate of the cells.

In this exemplary embodiment, it is understood that the following results may be obtained.

**[Table 1A]**

| gene type | survival of cells after addition of a compound in the presence of cell death inducing agent | | |
|---|---|---|---|
| SMS1(+/+), SMS2(+/+) | ×(Δ) | ○ | ○(Δ) |
| SMS1(-/-), SMS2(+/+) | × | × | ○ |
| SMS1(+/+) SMS2(-/-) | × | ○ | × |
| | ↑ SMSs inhibitor candidate | ↑ SMS2 selective inhibitor candidate | ↑ SMS1 selective inhibitor candidate |

| | | | |
|---|---|---|---|
| ○ : viable Δ : delay in cell growth × : death | | | |

As shown in the above Table, it can be determined that test compounds which cause death or delay in cell growth in cells expressing both SMS1 and SMS2 (SMS1(+/+) and SMS2(+/+)), and cause death in cells expressing SMS2 only (SMS1(-/-) and SMS2(+/+)) and cells expressing SMS1 only (SMS1(+/+) and (SMS2(-/-)) are candidates for inhibitor against SMSs. It can be determined that test compounds which allow survival of cells expressing both SMS1 and SMS2 and cells expressing SMS1 only, but cause death in cells expressing SMS2 only are candidates for SMS2 selective inhibitor, and test compounds which allow survival of cells expressing both SMS1 and SMS2 and cells expressing SMS2 only, but cause death in cells expressing SMS1 only are candidates for SMS1 selective inhibitor.

### (Screening kit for drugs)

In another aspect, the present invention provides kit for screening for a drug having at least one function selected from the group consisting of an anti-diabetes (such as drugs for type II diabetes mellitus, drugs ameliorating insulin-resistance (including insulin-resistance syndrome, Impaired Glucose Tolerance and the like)), an anti-obesity drug, a drug for decreasing visceral fat, a drug for treating fatty liver and a drug for increasing adiponectin expression, comprising a cell which only expresses either one gene of sphingomyelin synthase SMS1 or SMS2. Alternatively, the present invention provides kit for screening for a drug having at least one function selected from the group consisting of an anti-diabetes (such as drugs for type II diabetes mellitus, drugs ameliorating insulin-resistance (including insulin-resistance syndrome, Impaired Glucose Tolerance and the like)), an anti-obesity drug, a drug for decreasing visceral fat, a drug for treating fatty liver and a drug for increasing adiponectin expression, comprising a cell in which either one gene of sphingomyelin synthase SMS1 or SMS2 is knocked out and has at least one sphingomyelin synthase genetically introduced therein. The present kit is preferable in that it is applicable for allowing large scale by solving problems such as those it was difficult in a conventional method, in which fluorescently labeled ceramide (substrate for SMS) is incorporated into a cell, it is believed to be difficult to screen for a inhibitor in a large scale since operations such as lipid extraction and development with TLC and the like are carried out. In a case where cells are used in such an embodiment as this, there will be difference in growth capacity, which allows confirmation of presence or absence of inhibition in a plate reader by adding a reagent measuring growth, for example. As such, it is advantageous in that the present invention achieves "high through-put" in comparison to the conventional art. Furthermore, in a cell used in the present kit, any embodiment described in the Section (Methods for discovering a drug) described herein may be employed.

In one embodiment, the kit according to the present invention may be provided together with a cell death inducing agent such as cyclodextrin (for example, methyl beta cyclodextrin).

The kit according to the present invention may be associated with an instruction explicitly describing methods of use therefor. As used herein, "instruction" refers to what describes screening methods of the present invention and the like for users. Such an instruction describes instructions, for example, how cells according to the present invention are exposed to candidate substances or subject substances (for example, a library of organic low molecules), how cell death or delay in growth is measured by cell death inducing agent, and how evaluation should be performed. The instruction is optionally produced according to the format defined by the national controlling authority controlling the country where the present invention is performed. The instructions are usually provided as a package insert, and usually in a paper medium, but is not limited thereto, and for example, may be provided as a format such as an electronic medium (for example, website provided in the Internet, e-mails).

: Furthermore, in the instruction, determining methods may be described as those described in Section (Methods for discovering a drug) described herein.

### (Cells)

In another aspect, the present invention provides a cell in which at least either one gene of sphingomyelin synthase SMS1 or SMS2 is knocked out. Such a cell may be a cell in which only either one gene of sphingomyelin synthase SMS1 or SMS2 is knocked out. Cells in which either one gene of sphingomyelin synthase SMS1 or SMS2 is knocked out, may be used for screening for a substance which is effective for treating or preventing metabolic syndrome. Accordingly, the present invention provides a cell for screening for a substance effective for treating or preventing a metabolic syndrome, in which at least either one gene of sphingomyelin synthase SMS1 or SMS2 is knocked out (for example, a cell in which only either one gene of sphingomyelin synthase SMS1 or SMS2 is knocked out). Alternatively, the present invention provides a cell in which either one gene of sphingomyelin synthase SMS1 or SMS2 is knocked out wherein at least one sphingomyelin synthase is genetically introduced thereinto. Accordingly, a cell in which at least either one gene of sphingomyelin synthase SMS1 or SMS2 is knocked out, may be those in which either one gene of sphingomyelin synthase SMS1 or SMS2 is knocked out wherein at least one sphingomyelin synthase is genetically introduced thereinto. The cells according to the present invention employ any embodiments described in Section (Methods for discovering a drug) described herein.

### (General techniques)

Techniques of manufacturing a medical devise, techniques of formulation, techniques of microfabrication, molecular biological methods, biochemical methods, microbiological methods, saccharide chain related methods used in herein are well-known and routine in the art, and described in for example, Maniatis, T. et al.(1989) Molecular Cloning: A Laboratory Manual, Cold Spring Harbor and 3rd Ed. (2001); Ausubel, F. M., et al. eds, Current Protocols in Molecular Biology, John Wiley & Sons Inc., NY, 10158 (2000); Innis, M. A. (1990) PCR Protocols: A Guide to Methods and Applications, Academic Press; Innis, M. A. et al. (1995) PCR Strategies, Academic Press; Sninsky, J. J. et al. (1999) PCR Applications: Protocols for Functional Genomics, Academic Press; Gait, M. J. (1985) Oligonucleotide Synthesis: A Practical Approach, IRL Press; Gait, M. J. (1990) Oligonucleotide Synthesis: A Practical Approach, IRL Press; Eckstein, F. (1991) Oligonucleotides and Analogues: A Practical Approac, IRL Press; Adams, R. L. et al. (1992) The Biochemistry of the Nucleic Acids, Chapman & Hall; Shabarova, Z. et al. (1994) Advanced Organic Chemistry of Nucleic Acids, Weinheim; Blackburn, G. M. et al. (1996) Nucleic Acids in Chemistry and Biology, Oxford University Press; Hermanson, G. T. (1996) Bioconjugate Techniques, Academic Press; Method in Enzymology 230, 242, 247, Academic Press, 1994; Bessatsu Jikken Igaku [Experimental Medicine, Supplemental Volume], Idenshi Donyu Oyobi Hatsugen Kaiseki Jikken Ho [Experimental Methods for Transgenesis & Expression Analysis], Yodosha, 1997 or the like, relevant portions (that can be all) of which are incorporated herein by reference.

Methods of culture used in the present invention are described and supported in for example, Dobutsu Baiyo Saibo Manyuaru [Animal Cultured Cell Manual], Seno et al. ed., Kyoritsu Syuppan, 1993 or the like, the entire description of which are incorporated herein.

Hereinabove, the present invention has been described with showing preferred embodiments for easiness of understanding. While the present invention is described hereinbelow based on examples, the above description and the examples below are provided for illustrative purpose only, but not provided for a purpose for limiting the present invention. Therefore, the scope of the present invention is not limited by embodiments or examples that are specifically described herein, but limited only by the Claims.

### [Examples]

Handling animals used in the examples below were in accordance with the criteria defined in Hokkaido University.

Abbreviations used are as follows:
SMS: sphingomyelin synthetase
SMS1, SMS2: names of genes
KO: knockout
wKO: double knockout
MEF: mouse embryonic fibroblast
FBS: fetal bovine serum
DMEM: Dulbecco's modified Eagle medium
TG: triglyceride
SM: sphingomyelin
Me-beta-CD: methyl-beta-cyclodextrin
HFD: high fat diet
ND: normal diet
WAT: white fat cell mass
WT: wild-type

### (Example 1: Suppression of accumulation of neutral fat in the liver by suppressing SMS2)

### 1) Making SMSs knockout mice

As a mouse SMS, three isoforms have been found to date, SMS1 which was isolated by an expression cloning method, and SMS2 and SMSr which were identified as its homologs. SMS1 knockout (SMS1-KO) mice and SMS2 knockout (SMS2-KO) mice were both made by using the targeting vectors shown in Figure 1 in accordance with a general method for making a knockout mouse so as to make their exon 1 deficient.

### 2) Investigation of the effect of 60% fat diet administration on body weight

4 weeks old male wild-type mice (WT; C57BL6) and SMS2-KO mice were administered a high fat diet (HFD; 58Y1, testDiet) and a control normal diet (ND; AIN76A, testDiet), respectively, and change in body weight were measured for 9 weeks. As a result, as shown in Figure 2, when SMS2 KO mice were fed by HFD, increase in body weight was significantly suppressed compared to when similar HFD was administered to WT.

### 3) Investigation of the effect of 60% fat diet administration on white fat cell mass (WAT)

WT and SMS2 KO mice to which HFD were administered similarly to 2) (12 weeks later) were subjected to abdominal section, and fat attached to epididymis was obtained and then the weights were measured. As a result, as shown in Figure 3, in SMS2 KO/HFD, white fat cell mass (WAT) appeared to tend to decrease, which was not a significant difference. Although not shown in the Figure, a similar experiment wherein a control normal diet (ND) was fed in parallel was also carried out. In the latter experiments, white fat cell mass (WAT) was decreased in the SMS2 KO/HFD group compared to the WT/HFD group, on the other hand, any significant difference was not found between the SMS2 KO/HFD group and the SMS2 KO/ND group.

### 4) Investigation of the effect of 60% fat diet administration on liver triglyceride amount

WT and SMS2 KO mice to which HFD were administered similarly to 2) (12 weeks later) were subjected to abdominal section, and the livers thereof were obtained. Triglyceride in the liver homogenate was quantified by Triglyceride Quantification Kit (Biovision). The methods were in accordance with the written instructions of the kit. The protein amount in the homogenate was quantified with BCA protein assay (Pierce) and used for correction of triglyceride amounts. As a result, as shown in **Figure 4**, compared to the WT/HFD group, the triglyceride amount was significantly decreased in the SMS2 KO/HFD group.

Furthermore, the result of comparing triglyceride amount in liver homogenate (mg/mg protein) is shown in **Figure 4A** between wild-type (WT) and SMS2-KO mice to which a high fat diet (HFD; 60% fat diet) or a control normal diet (ND) were administered for 12 weeks. As a result, as shown in **Figure 4A****,** compared to the WT/HFD group (left), the triglyceride amount was significantly decreased in the SMS2 KO/HFD group (right). On the other hand, any significant difference was not found between the SMS2 KO/HFD group and the SMS2 KO/ND group.

### 5) Investigation of the effect of 60% fat diet administration on adiponectin amount

WT and SMS2 KO mice to which HFD were administered similarly to 2) (12 weeks later) were subjected to abdominal section, and fat attached to epididymis was obtained, mRNA thereof was extracted and then the mRNA expression amount of adiponectin was measured by QPCR (Thermal Cycler Dice TP800, TAKARA BIO INC.). At that time, correction was made with the mRNA expression amount of GAPDH in the same samples. As reagents, PrimeScript RT-reagent Kit (TAKARA BIO INC.) was used for the first strand cDNA synthesis and SYBR premix EX Taq II (TAKARA BIO INC.) was used for QPCR. As PCR primers,
Fw primer: GTCAGTGGATCTGACGACACCAA (**SEQ ID NO: 1**);
Rv primer: ATGCCTGCCATCCAACCTG (**SEQ ID NO: 2**) were used.

As a result, as shown in **Figure 5****,** the expression of adiponectin was suppressed in the WT/HFD group, while a relatively high expression amount was maintained in the SMS2 KO/HFD group. Furthermore, relative expression amount of adiponectin was compared between wild-type (WT) and SMS2-KO mice to which a high fat diet (HFD; 60% fat diet) or a control normal diet (ND) was administered for 12 weeks. As a result, as shown in **Figure 5A**, in the WT group, by feeding the high fat diet, the expression of adiponectin was suppressed, while in the SMS2 KO group, even when the high fat diet was fed, a relatively high expression amount was maintained.

From the above results, it was found that there was possibility that the accumulation of neutral fat in the liver is suppressed by suppressing SMS2.

### 6) Experiments regarding to insulin resistance

### 6-1) Expression analysis of an insulin receptor

Next, mRNA expression amount of an insulin receptor in adipose tissues in WT and SMS2KO mice was analyzed by a quantitative PCR. The relative expression amount of insulin receptor was investigated when wild-type (WT) and SMS2-KO mice were administered a high fat diet (HFD; 60% fat diet) or a control normal diet (ND). The primer sequence and control primer sequence used are shown. The quantitative PCR was carried out in the same way as described in 5).
Insulin receptor (IR)
Forward (Fw): CAGCTCGAAACTGCATGGTTG (**SEQ ID NO: 3**)
Reverse (Rv): GGTGACATCCACCTCACAGGAA (**SEQ ID NO: 4**)

The result is shown in **Figure 5B-1****.** As shown in **Figure 5B-1**, in the wild-type mice, when a high fat diet (HFD; 60% fat diet) was fed, the expression amount of insulin receptor decreased. On the other hand, in the SMS2-KO mice, decrease in the expression of insulin receptor by the high fat diet did not occur and it was an almost similar expression amount to that of a case which the control normal diet was fed. In addition, the expression amount of insulin receptor when the high fat diet was administered to the SMS2-KO mice was significantly higher compared to when the high fat diet was administered to the wild-type mice.

From above, in the SMS2-KO mice, decrease in expression of an insulin receptor by HFD as observed in the wild-type did not occur and the expression amount was almost similar to that with ND. From the above results, it was suggested that in adipose tissues of the SMS2-KO mice, decrease in the expression of insulin receptor by HFD as observed in the WT did not occur, and thus the SMS2-KO mice did not become insulin resistance (**Figure 5B-1**).

### 6-2) Expression analysis of Glut4

Next, the effect of 60% fat diet administration on Glut4 in adipose tissues was investigated. The relative expression amount of Glut4 was compared between a wild-type (WT) and SMS2-KO mice to which a high fat diet (HFD; 60% fat diet) or a control normal diet (ND) were administered. The primer sequences and control primer sequences used are shown.
GLUT4:
Forward (Fw): CTGTAACTTCATTGTCGGCATGG (SEQ ID NO: 5)
Reverse (Rv): AGGCAGCTGAGATCTGGTCAAAC (SEQ ID NO: 6)
Hypoxanthine-guanine phosphoribosyltransferase (HPRT) that is an endogenous control gene: Forward (Fw): TTGTTGTTGGATATGCCCTTGACTA (SEQ ID NO: 7)
Reverse (Rv): AGGCAGATGGCCACAGGACTA (SEQ ID NO: 8).

The result is shown in **Figure 5B-2**. As apparent from **Figure 5B-2**, In the wild-type mice, when a high fat diet (HFD; 60% fat diet) was fed, the expression amount of Glut4 was decreased, while in the SMS2-KO mice, the degree of decrease in the expression of Glut4 by a high fat diet was suppressed and it was a almost similar expression amount to that of a case which a control normal diet was fed. In addition, the expression amount of Glut4 when the high fat diet was administered to the SMS2-KO mice was significantly higher compared to when the high fat diet was administered to the wild-type. From the above results, it was suggested that in adipose tissues of the SMS2-KO mice, decrease in the expression of Glut4 by the high fat diet as observed in the wild-type mice did not occur, and thus the SMS2-KO mice did not become insulin resistance.

### 6-3) Glucose tolerance test

The control normal diet (ND) or high fat diet (HFD; 60% fat diet) described in "2) Investigation of the effect of 60% fat diet administration on body weight" above were administered to wild-type (WT) or SMS2KO mice. The mice which were further grown for 9 weeks were used for carrying out glucose tolerance test. The mice were fasted for 24 hours and intraperitoneally administered 2g/kg of glucose. Blood glucose levels were measured in the blood obtained from tail vein immediately before administration, 15 minutes after administration, at 30 minutes after administration, at 60 minutes after administration, and at 120 minutes after administration. For the measurement, a glucometer was used. As a result, it was found between the wild-type and SMS2-KO mice to which the high fat diet was administered, that at 120 minutes after administration, the blood glucose level was significantly decreased in the SMS2 KO/HFD group (P<0.05; Figure 5C). When the areas under curve of the graph of the glucose tolerance test were compared (GTT AUC; Figure 5D), between the wild-type and the SMS2-KO mice to which the high fat diet was administered, the area was significantly decreased in the SMS2-KO mice (P<0.05).

**Figure 5D** shows the area under curve (AUC) of Figure 5C as a bar graph. Also from such expression, it can be understood that blood glucose level was significantly decreased in the SMS2 KO/HFD group compared to the WT/HFD group.

From the results, it could be confirmed that they become significantly sensitive to glucose load.

Next, plasma insulin amount was measured. Wild-type (WT) and SMS2-KO mice were administered a normal diet from the time of 8 weeks old for 4 weeks, and then fasted for 24 hours, and then administered a normal diet. Blood insulin concentration was measured at 24 hours fasting and 1 hour after the normal diet administration. The results are shown in Figure 5E. As apparent from Figure 5E, between the wild-type (WT) and SMS2-KO mice, no significant difference in blood insulin concentration was observed at 24 hours fasting and 1 hour after the normal diet administration. Therefore, it can be said that no significant difference in blood insulin concentration and reactivity was observed between the wild-type (WT) and SMS2-KO mice. From the results, it could be confirmed that the SMS2-KO mice did not have any abnormality in insulin producing capability and reactivity to glucose, and thus any abnormality in pancreatic beta cell was observed.

Next, the effect of a high fat diet on blood glucose level was investigated. 4 weeks old wild-type (WT) and SMS2-KO mice were administered a high fat diet (HFD; 60% fat diet) for 2 weeks. Blood glucose level was measured at the time of 4 hours fasting before the high fat diet administration and at the time of 4 hours fasting after 2 weeks of high fat diet administration. As shown in **Figure 5F****,** although no significant difference in blood glucose level was observed before administering the high fat diet between the wild-type mice and SMS2-KO mice, blood glucose level in the wild-type mice was significantly higher compared to in the SMS2-KO mice after administering the high fat diet for 2 weeks.

Next, the effect of a high fat diet on blood insulin amount was investigated. Wild-type (WT) and SMS2-KO mice were administered a high fat diet (HFD; 60% fat diet) for 2 weeks, then fasted for 4 hours, and then blood insulin amount was measured. As a result, as shown in **Figure 5G**, in the SMS2-KO mice, blood insulin amount was decreased with a significant difference compared to in the wild-type mice.

Next, the blood glucose concentration in response to insulin was investigated. 4 weeks old wild-type (WT) and SMS2-KO mice were administered a high fat diet (HFD; 60% fat diet) for 6 weeks, and insulin was intraperitoneally administered in 0.5U/kg. The results of blood glucose concentrations that were measured before administration and at 15, 30, 60, 90 and 120 minutes after administration are shown in **Figure 5H-1**. As shown in **Figure 5H-1****,** in particular, since blood glucose concentration in the SMS2-KO mice was significantly decreased compared to in the wild-type mice at 30, 60 and 90 minutes after administration, it became apparent that the SMS2-KO mice were more sensitive to insulin.

As also apparent from **Figure 5H-2** which shows the area under curve (AUC) in **Figure 5H-1**, since it was significantly decreased by about 20% in the SMS2-KO mice compared to in the wild-type mice, it became apparent that they were more sensitive to insulin.

In the SMS2-KO mice, plasma insulin amount was decreased compared to the WT. It was thus demonstrated that the SMS2KO mice did not become insulin resistance. Therefore, the SMS2-KO mice was sensitive to insulin, and insulin resistance was not raised therein as in the wild-type mice (see, in particular, **Figure 5E****,** **Figure 5F** and **Figure 5G**).

From the above results, it could be found that there is possibility that diabetes can be treated, in particular, insulin resistance can be ameliorated, by inhibiting SMS2.

### (Example 2: Production of SMSs-reconstructed cells)

### 1) Production of immortalized MEF cells

As described in Shin Baiyo Saibo Jikken Ho [New Cultured Cell Experimental Method], Yodosha Co., Ltd., Chapter 4, Shodai Baiyo Sen'iga Saibo [A Primary Cultured Fibroblast], p.66-70, the SMS1-KO mouse and SMS2-KO mouse made in Example 1 were mated and from the thus obtained fetus of double knockout that were deficient in both SMS1 and SMS2 (SMS1,2-wKO), an MEF (mouse embryonic fibroblast) was isolated. The MEF was infected with a retrovirus that was released into the culture supernatant of 293T cell stably retaining pMFG-SV40Tst, thereby immortalizing MEF.

### a) Preparation of a viral solution

As a virus producing cell, NIH3T3 ecotroic producer cell psiCRE-MFGtsT (RCB1119; SV40 T antigen-expressing virus producer cell; see, http://www2.brc.riken.jp/lab/cell/detail.cgi?cell_no=RCB111 9&type=1) was used. The psiCRE-MFGtsT cell releases a retrovirus expressing SV40tsT into a medium and it infects a mouse host cell. A subconfluent psiCRE-MFGtsT cell was cultured in a DMEM including 10% FBS overnight under conditions of 37 degrees Celsius, 5% CO₂. At the next day, the supernatant was recovered as a viral solution and filtered with a 0.20 mm syringe filter (available from SCORNING).

### b) Viral infection and cloning of cell strains

In the culture of MEF, the medium was replaced with 5mL of DMEM including 10% FBS, 1 mL of the viral solution, it was cultured for 24 hours under conditions of 34 degrees Celsius, 5% CO₂ and infected with the virus, then added 5 mL of DMEM including 10% FBS and further cultured overnight. At the next day, the medium was replaced with 10 mL of DMEM including 10% FBS. The medium was exchanged every 2-3 days, and immortalized MEF cell strains were established. Furthermore, some cell strains were cloned from the immortalized MEF and one of them was designated as ZS2.

### 2) Production of immortalized MEF cells that overexpress SMS1 or SMS2

A retroviral vector was made by incorporating SMS1 and SMS2 with a V5 tag at C terminus added into pQCXIP (Clontech). Using this plasmid and GP2-293 cell (Clontech), retroviruses expressing SMS1 or SMS2, respectively were made and, in accordance with the instructions of Clontech, infected into the ZS2 cell that was one of the cell strains cloned in 1). Uninfected cells were removed with 4 microgram/ml puromycin, cell ZS2/SMS1 that overexpressed SMS1 and cell strain ZS2/SMS2 that overexpressed SMS2 were obtained. On three these cell strains, a Western blot was carried out with an anti-V5 mab (invitrogen) antibody. In the SMS1 expressing cell and the SMS2 expressing cell, as shown in Figure 6, bands of SMS1 or SMS2 which can be detected with the anti-V5 mab were respectively confirmed.

### (Example 3: Investigation of function of ZS2, ZS2/SMS1 and ZS2/SMS2 cells)

The function, in particular, SMS activity and TG synthesis, of the ZS2, ZS2/SMS1 and ZS2/SMS2 cells made in Example 2.

### 1) An SMS activity

The cells made in Example 2 were spread in 6-well plates each (2x10⁵ cell/well), were cultured overnight in DMEM including 10% FBS, then the mediums were replaced with DMEM without serum, and BODIPY-C5-ceramide (molecular probe) adjusted to a final concentration of 1 micromolar was added and they were allowed to react at 37 degrees Celsius for 30 minutes. After the cells were detached by pipetting, they were solubilized with 100 microliter of a solubilizing buffer (20mM Tris-HCl, pH7.5, 0.2% Triton X-100, 1 x complete (Roche), 1 mM PMSF) and the protein amounts were measured with BCA protein assay (Pierce). Lipids were extracted from the solubilized solution with Bligh & Dyer method, and using HPTLC (Merck), BODIPY-ceramide and the produced BODIPY-C5-sphingomyelin were quantified with Fla7000 (Fuji film) and the SMS activity was calculated. The SMS activity was corrected for protein amount. As shown in Figure 7, ZS2/SMS1 cell and ZS2/SMS2 cell had an SMS activity, while ZS2 cell did not have any SMS activity.

### 2) TG synthesis

The cells made in Example 2 were spread into 6-well plates (2x14⁵ cell/plate), cultured overnight in DMEM with 10% FBS, then the mediums were replaced with DMEM without serum, and [¹⁴C] oleic acid (American Radiolabeled Chemicals) at a final concentration of 1 microcurie was added and allowed to react at 37 degrees Celsius for 30 minutes. After the cells were detached by pipetting, they were solubilized with 100 microliter of a solubilizing buffer (20 mM Tris-HCl, pH 7.5, 0.2% Triton X-100, 1 x complete (Roche), 1 mM PMSF) and the protein amounts were measured with BCA protein assay (Pierce). Lipids were extracted from the solubized solution with Bligh & Dyer method, and using HPTLC (Merck), [¹⁴C] oleic acid and the produced [¹⁴C] oleic acid-triglyceride were quantified with Fla7000 (Fuji film) and the TG amounts were calculated. As shown in Figure 8, there was a significant difference between the TG amount in the ZS2/SMS1 cell and the ZS2/SMS2 cell and the TG mount of the ZS2 cell (p<0.005 and p<0.02, respectively).

### (Example 4: Development of methods for searching a SMS inhibitor using MEF derived from SMS1,2-wKOmouse)

Since in a cell in which the amount of SM is reduced, cell death can be induced by cyclodextrin (see Yamaoka et al., *supra*, Fig. 2D), it is believed that ZS2/SMS1,2 cells, ZS2/SMS2 cells and ZS2/SMS1 cells are cultured in the presence of MeCD, and a variety of test compounds are added to the culture, and an assay may be performed relating to cell survival rate and growth rate to allow search for inhibitor.

Assays relating to cellular survival and growth rate are conducted according to NPL 1. Specifically, 1 x 10⁶ MEF cells are washed, and resuspended in 1 ml of serum-free RPMI 1640 medium, and treated with an appropriate concentration of MebetaCD, and cultured in 5% CO₂, 37degrees Celsius for five minutes. After 2 ml of normal medium containing a variety of compounds are added thereto, and the cells are cultured for additional 12 hours. A reagent for measuring viable cell number containing WST-8 (Nakalai Tesque) may be used to measure survival rate of the cells.

Alternatively, a different method may be performed. In the present example, cells to be evaluated were seeded in 48-well plate dish at 5000 cells/0.2ml/well. For cell culture, 0.3% FCS/OPTIPRO SFM (GIBCO) was used. MebetaCD was added at the final concentration of 0-7mum, and further cultured in 5% CO₂ 37degrees Celsius for sixteen hours. A reagent for measuring viable cell number containing WST-8 (DOJINDO) was added at 10microliter/well and further cultured in 5% CO₂, 37degrees Celsius for one to six hours, and measured the viable cell numbers according to the protocol.

**[Table 1B]**

| **cells** | **gene type** | survival of MEF after addition of a compound in the presence of MebetaCD | | |
|---|---|---|---|---|
| ZS2/SMS1,2 | SMS1(+/+). SMS2(+/+) | ×(Δ) | ○ | ○(Δ) |
| ZS2/SMS2 | SMS1(-/-), SMS2(+/+) | × | × | ○ |
| ZS2/SMS1 | SMS1(+/+) SMS2(-/-) | × | ○ | × |
| | | ↑ SMSs inhibitor candidate | ↑ SMS2 selective inhibitor candidate | ↑ SMS1 selective inhibitor candidate |

| | | | | |
|---|---|---|---|---|
| ○:viable Δ:delay in cell growth ×:death | | | | |

As shown in the above Table, test compounds which cause death or delay in cell growth in cells expressing both SMS1 and SMS2 (SMS1(+/+) and SMS2 (+/+)), and cause death in cells expressing SMS2 only (SMS1(-/-) and SMS2(+/+)) and cells expressing SMS1 only (SMS1(+/+) and (SMS2(-/-)) are candidates for inhibitor against SMSs. It can be determined that test compounds which allow survival of cells expressing both SMS1 and SMS2 and cells expressing SMS1 only, but cause death in cells expressing SMS2 only are candidates for SMS2 selective inhibitor, and test compounds which allow survival of cells expressing both SMS1 and SMS2 and cells expressing SMS2 only, but cause death in cells expressing SMS1 only are candidates for SMS1 selective inhibitor.

### (Example 5, confirmation of the method of search for SMS inhibitor using MEF derived from SMS1,2-wKO mouse)

Myriocin (ISP-1, Enzo Life Sciences, Cat. No. SL-226), which is known to inhibit synthesis of SM precursor, and thus results in reduction the amount of SM in cell, is used to confirm whether the method of search according to Example 4 does function.

1 x 10⁶ MEF cells are washed, and resuspended in 1 ml of serum-free RPMI 1640 medium, and treated with an appropriate concentration of Me beta CD, and cultured in 5% CO₂, 37degrees Celsius for five minutes. After 2 ml of normal medium containing myriocin are added thereto, and the cells are cultured for additional 12 hours. A reagent for measuring viable cell number containing WST-8 (Nakalai Tesque) may be used to measure survival rate of the cells.

More specifically, ZS2/SMS2 cells prepared in Example 2 are placed in 48-swell plate dish at 5000 cells/0.2ml/well (N=3). For cell culture, 0.3% FCS/OPTIPRO SFM (GIBCO) was used. In this case, Myriocin (Final concentration 5microM) is added, or alternatively, Myriocin + ceramide (10microM) or Myriocin+sphingomyelin (15microM) were added thereto. Me beta CD was added at the final concentration of 2mM, and further cultured in 5% CO₂ 37degrees Celsius for sixteen hours. A reagent for measuring viable cell number containing WST-8 (DOJINDO) was added at 10microliter/well and further cultured in 5% CO₂, 37degrees Celsius for one hour (WST assay). The viable cell numbers were counted according to the protocol. The results are shown in Figure 9. Y axis represents a numerical value when the average value is deemed to be 100% amongst the cases in the presence and absence of Myriocin, when Me beta CD is not placed therein. There was not significant difference between the presence and absence of Myriocin, and thus the average of these has been made to be 100 %. The results are shown in Figure 9.

As shown in Figure 9, although significant difference was found in survival rate when Myriocin is added to the cells (two left-hand ones), no significant difference was found in survival rate by Myriocin when ceramide (Cer) or sphingomyelin (SM) was added to the medium. Therefore, it has been confirmed that the reduction of sphingomyelin by Myriocin increased sensitivity to Me beta CD sensitivity.

Consequently, in ZS2/SMS2 cells, cell growth was reduced by about 50 % by the addition of 2mM Me beta CD, and the addition of Myriocin (2mM) further reduced the cell growth by about 20 %. In this case, the addition of ceramide or SM almost completely inhibited the cell growth inhibitory effects caused by Myriocin, and the viable cell number was returned to the viable cell number found for those no Myriocin added thereto (with respect to test for significance, all samples show p<0.01). Based on the above-mentioned results, in the subject system, it was demonstrated that inhibitors of SM synthesis pathway such as Myriocin reduced the viable cell number, and further such an effect is reverted by addition of Ceramide or SM to the medium. Therefore, it was demonstrated that the inhibition of SM synthesis caused cell death. Accordingly, it was demonstrated that the subject system can be used to isolate inhibitors for enzymes relating to a series of SM synthesis pathway using cell death as indicative therefor.

As such, it has been shown that the method of search described in Example 4 is a useful method of search for SMS inhibitors.

Next, the subject search system is used to demonstrate that the effects attained as described in Example 4 may be achieved, i.e., that Me beta CD induces cell death in ZS2 cells, but not in ZS2 cells with SMS genetically introduced thereinto.

ZS2, ZS2/SMS1,2 ZS2/SMS1 or ZS2/SMS2 cells are placed in 48-well plate dish at 5000 cells/0.2ml/well (N=3). For cell culture, 0.3% FCS/OPTIPRO SFM (GIBCO) is used. Me beta CD was added at the final concentration of 5 mM, and further cultured in 5% CO₂, 37degrees Celsius for sixteen hours. A reagent for measuring viable cell number containing WST-8 (DOJINDO) was added at 10 microliter/well and further cultured in 5% CO₂, 37degrees Celsius for one to six hours, and measured the viable cell numbers according to the protocol.

Consequently, it was demonstrated that in comparison to ZS2 cells, ZS2/SMS1,2 ZS2/SMS1 and ZS2/SMS2 cells are all increased cell growth by 70% to 90%, and thus these cells became resistant to cell death caused by Me beta CD (regarding test for significance, all are p<0.01) (Figures 10-12). Figure 10 shows the comparison between ZS2/SMS1,2 and ZS2 cells, Figure 11 shows the comparison between ZS2/SMS2 and ZS2 cells, and Figure 12 shows the comparison between ZS2/SMS1 and ZS2 cells.

Next, it is verified whether SMS1 or SMS2 specific inhibitors may be actually isolated using the subject screening system. Specifically, siRNA against the respective SMSs are used as SMSs specific inhibitors, and the above-mentioned Me beta CD sensitive tests are performed, and then the respective siRNA can cause cell death only in the case where only the SMSs of interest is/are knocked down, thereby proving that such a specific inhibitors can be isolated.

: Sequence information of double stranded siRNAs against SMS1 or SMS2 used are listed below:
Sense strand DNA sequence corresponding to siRNA SMS1-i4 specific to mouse SMS1:
5'-ggaattgtacctcgatctta-3'(SEQ ID NO: 9)
Sense strand DNA sequence corresponding to siRNA SMS2-i11 specific to mouse SMS2:
5'-ggctcttctgcgttacaa-3'(SEQ ID NO: 10)

Sense strand DNA sequence corresponding to siRNA CTR-i used as a control:
5'-ttctccgaacgtgtcacgt-3'(SEQ ID NO: 11)

The respective primer sequences used for detecting mouse G3PDH, SMS1 or SMS2 by quantitative PCR:
G3PDH
Forward (Fw) :5'-caactcccactcttccaccttc-3'(SEQ ID NO: 12)
Reverse (Rv):5'-cttactccttggaggccatgtag-3'(SEQ ID NO: 13)
SMS1
Forward (Fw):5'-tacactgtggacgtggtgg -3'(SEQ ID NO: 14)
Reverse (Rv):5'-gggccaatggtaagatcga -3'(SEQ ID NO: 15)
SMS2
Forward (Fw):5'-tcaatggagactctcaggc -3'(SEQ ID NO: 16)
Reverse (Rv):5'- ccgctgaagaggaagtctc-3'(SEQ ID NO: 17)

Hepa1c1c7 cells (ATCC), a murine hepatic parenchymal cell line, and ZS2/SMS1 or ZS2/SMS2 cells were used to evaluate RNAi activity of siRNA. The cells were placed in 12 well dish at 1 x 10⁴ cells. After twenty-four hours, commercially available transforming reagent RNAiMAX (Invitrogen) is used to conduct transformation according to the manufacturer. The final concentration of the siRNAs used was 100nM. After forty-eight hours, the cells were lysed with 0.5ml Trizol (Invitrogen), and RNA extracted therefrom according to the protocol. The reverse transcription into cDNA was conducted using Superscript III (Invitrogen), a commercially available kit according to a routine method. Quantitative PCR was performed using the above-mentioned primers and a commercially available kit (ABI).

Consequently, SMS1-i4, a siRNA against SMS1 knocked down expression of SMS1 by 90% or greater (Figure 13). Similarly, SMS2-i11, a siRNA against SMS2 knocked down expression of SMS2 by 90% or greater (Figure 14). Furthermore, in the case where ZS2/SMS2 cells were used, it was also confirmed that the expression of SMS2 was knocked down by about 80%, in a similar manner (Figure 15). In Figures 13 through 15, comparative data of expression amount are shown in which the amount of cDNA between samples was corrected according to the amount of expression of G3PDH as an internal control. Additionally, it was also confirmed that in Figure 13, the expression of SMS2 was not expressed, and in Figure 14, the expression of SMS1 was not expressed. As such, specificity have also been confirmed in the mRNA level.

To 6-well dishes, 2 x 10⁵ cells of ZS2/SMS1 or ZS2/SMS2 cells were placed in suspension onto 2ml 10% FCS/DMEM medium. A variety of siRNAs were added thereto after mixing with a commercially available transformation reagent at the final concentration of 100nM. The transformation reagent used is RNAiMAX (Invitrogen) and transformation was performed according to the manufacturer's protocol. Thereafter, the medium was replace with 0.3%FCS/OPTIPRO SFM(GIBCO) and the cells were placed on 48-well dish in an equal manner. The amount of medium is 200µL/well. After culturing for additional twenty four hours, Me beta CD is added at the final concentration of 5mM, and cultured in 5% CO₂ 37 degrees Celsius for sixteen hours. A reagent for measuring viable cell number containing WST-8 (DOJINDO) was added at 10microliter/well and further cultured in 5% CO₂, 37degrees Celsius for one to six hours, and measured the viable cell numbers according to the protocol.

When SMS2 is knocked down in ZS2/SMS2 cells, the cell growth became sensitive to ME beta CD and was reduced to about 40% (with respect to test for significance, all samples show p<0.01) (Figure 18). However, when SMS2 were knocked down in ZS2/SMS1 cells, no change was found in ME beta CD sensitivity (Figure 19). Similarly, when SMS1 is knocked down in ZS2/SMS1 cells, the cell growth became sensitive and was reduced to about 20% (with respect to test for significance, all samples show p<0,01) (Figure 16). However, when SMS2 were knocked down in ZS2/SMS1 cells, no change was found in ME beta CD sensitivity (Figure 17). As such, with respect to the experimental results using ZS2/SMS2 cells, by specifically knocking down SMS2 in SMS2 expressing cells, it becomes sensitive to ME beta CD. Therefore, the effects of siRNA are dependent on the expression of SMS2 and thus off target effects have been able to be eliminated. Furthermore, as a method for isolating SMS2 specific inhibitors, it has been confirmed that SMS2 specific inhibitors can be isolated by screening for compounds which ZS2/SMS2 cells become ME beta CD sensitive, and ZS2/SMS1 cells do not become ME beta CD sensitive.

while, as described above, the present invention has been illustrated with preferred embodiments of the present invention, it is understood that the scope of the present invention should be interpreted only by the Claims. It is understood that the content of the patents, patent applications and literatures referred herein should be incorporated herein for reference same as if the content thereof itself is specifically described herein.

The present application claims priority to Japanese Patent Application No. 2009-219751. It is understood that the entire content thereof is to be incorporated as constituting a part of the present specification in a similar manner to as if it is specifically described herein.

### [Industrial Applicability]

The present invention provides a method for screening for a drug for treating and preventing a metabolic disease.

### [Sequence Listing Free Text]

SEQ ID NO: 1: a Forward(Fw) primer for adiponectin used in Example 1
SEQ ID NO: 2: a Reverse(Rv) primer for adiponectin used in Example 1
SEQ ID NO: 3: a Fw primer for insulin receptor used in Example 1
SEQ ID NO: 4: a Rv primer for insulin receptor used in Example 1
SEQ ID NO: 5: a Fw primer for GLUT4 used in Example 1
SEQ ID NO: 6: a Rv primer for GLUT4 used in Example 1
SEQ ID NO: 7: a Fw primer for internal control gene (HPRT) used in Example 1
SEQ ID NO: 8: a Rv primer for internal control gene (HPRT) used in Example 1
SEQ ID NO: 9: sense strand DNA sequence corresponding to siRNA SMS1-i4
SEQ ID NO: 10: sense strand DNA sequence corresponding to siRNA SMS2-i11
SEQ ID NO: 11: sense strand DNA sequence corresponding to siRNA CRT-i
SEQ ID NO: 12: PCR Forward (Fw) primer for detecting mouse G3PDH mRNA
SEQ ID NO: 13: PCR Reverse (Rv) primer for detecting mouse G3PDH mRNA
SEQ ID NO: 14: PCR, Forward (Fw) primer for detecting mouse SMS1 mRNA
SEQ ID NO: 15: PCR Reverse (Rv) primer for detecting mouse SMS1 mRNA
SEQ ID NO: 16: PCR Forward (Fw) primer for detecting mouse SMS2 mRNA
SEQ ID NO: 17: PCR Reverse (Rv) primer for detecting mouse SMS2 mRNA

## Claims

1. A method for screening for a substance effective for treating or preventing a metabolic syndrome, comprising the step of measuring inhibitory activity of a subject substance against sphingomyelin synthase, wherein when the subject substance has an inhibitory activity of a sphingomyelin synthase, the subject substance is determined to be effective for treating or preventing the metabolic syndrome.

2. The method according to claim 1, wherein the substance effective for treating or preventing the metabolic syndrome comprises at least one function selected from the group consisting of an anti-diabetes, an anti-obesity drug, a drug for decreasing visceral fat, a drug for treating fatty liver and a drug for increasing adiponectin expression.

3. The method according to claim 1, wherein the measurement of the inhibitory activity is carried out using a cell in which either one gene of sphingomyelin synthase SMS1 or SMS2 is knocked out.

4. The method according to claim 1, wherein the measurement of the inhibitory activity is carried out using a cell in which either one gene of sphingomyelin synthase SMS1 or SMS2 is knocked out and has at least one sphingomyelin synthase genetically introduced therein.

5. The method according to claim 3 or 4, wherein when the cell is given cyclodextrin and the subject substance causes delay in cell growth or causes cell death, the subject substance is determined to have said at least one function.

6. The method according to claim 3 or 4, wherein said at least one sphingomyelin synthase is SMS1 or SMS2, or both SMS1 and SMS2.

7. The method according to claim 3 or 4, wherein said at least one sphingomyelin synthase is SMS2.

8. A kit for screening for a substance effective for treating or preventing a metabolic syndrome, comprising a cell in which either one gene of sphingomyelin synthase SMS1 or SMS2 is knocked out.

9. A kit for screening for a substance effective for treating or preventing a metabolic syndrome, comprising a cell in which either one gene of sphingomyelin synthase SMS1 or SMS2 is knocked out and has at least one sphingomyelin synthase genetically introduced therein.

10. The kit according to claim 8 or 9, said substance comprises at least one function selected from the group consisting of an anti-diabetes, an anti-obesity drug, a drug for decreasing visceral fat, a drug for treating fatty liver and a drug for increasing adiponectin expression

11. The kit according to claim 10, further comprising cyclodextrin.

12. The kit according to claim 8 or 9, wherein said at least one sphingomyelin synthase is SMS1 or SMS2, or both SMS1 and SMS2.

13. The kit according to claim 8 or 9, wherein said at least one sphingomyelin synthase is SMS2.

14. A cell for screening for a substance effective for treating or preventing a metabolic syndrome, in which either one gene of sphingomyelin synthase SMS1 or SMS2 is knocked out.

15. A cell in which sphingomyelin synthase SMS1 and SMS2 have been knocked out wherein at least one sphingomyelin synthase is genetically introduced thereinto.


SEQUENCE LISTING

<110>  National University Corporation Hokkaido University
       Shionogi Co., LTD.
       Susumu, MITSUTAKE
       Yasuyuki, IGARASHI
       Kota, ZAMA
       Tetsuya, YOSHIDA
       Yoshikazu, TANAKA
       Hiroshi, TAKEMOTO

<120>  Amelioration of metabolic syndrome using physiological functions
       of sphingomyelin synthase SMS2, or screening methods for
       ameliorating agents

<130>  U1477 EP S3

<140>  EP 10 81 8555.4
<141>  2010-09-22

<150>  JP 2009-219751
<151>  2009-09-24

<160>  17

<170>  PatentIn version 3.3

<210>  1
<211>  23
<212>  DNA
<213>  Artificial

<220>
<223>  Fw primer for Adiponectin

<400>  1
gtcagtggat ctgacgacac caa                                              23


<210>  2
<211>  19
<212>  DNA
<213>  Artificial

<220>
<223>  Rv primer  for Adiponectin

<400>  2
atgcctgcca tccaacctg                                                   19


<210>  3
<211>  21
<212>  DNA
<213>  Artificial

<220>
<223>  Fw primer for insulin receptor

<400>  3
cagctcgaaa ctgcatggtt g                                                21


<210>  4
<211>  22

<212>  DNA
<213>  Artificial

<220>
<223>  Rv primer for insulin receptor

<400>  4
ggtgacatcc acctcacagg aa                                                22


<210>  5
<211>  23
<212>  DNA
<213>  Artificial

<220>
<223>  Fw primer for GLUT4

<400>  5
ctgtaacttc attgtcggca tgg                                               23


<210>  6
<211>  23
<212>  DNA
<213>  Artificial

<220>
<223>  Rv primer for GLUT4

<400>  6
aggcagctga gatctggtca aac                                               23


<210>  7
<211>  25
<212>  DNA
<213>  Artificial

<220>
<223>  Fw primer for HPRT

<400>  7
ttgttgttgg atatgccctt gacta                                             25


<210>  8
<211>  21
<212>  DNA
<213>  Artificial

<220>
<223>  Rv primer for HPRT

<400>  8
aggcagatgg ccacaggact a                                                 21


<210>  9
<211>  20
<212>  DNA
<213>  Artificial

<220>
<223>  sense DNA sequence corresponding to siRNA SMS1-i4

<400>  9
ggaattgtac ctcgatctta                                                   20


<210>  10
<211>  18
<212>  DNA
<213>  Artificial

<220>
<223>  sense DNA sequence corresponding to siRNA SMS2-i11

<400>  10
ggctcttctg cgttacaa                                                     18


<210>  11
<211>  19
<212>  DNA
<213>  Artificial

<220>
<223>  sense DNA sequence corresponding to siRNA CTR-i

<400>  11
ttctccgaac gtgtcacgt                                                    19


<210>  12
<211>  22
<212>  DNA
<213>  Artificial

<220>
<223>  Fw primer for G3PDH

<400>  12
caactcccac tcttccacct tc                                                22


<210>  13
<211>  23
<212>  DNA
<213>  Artificial

<220>
<223>  Rv primer for G3PDH

<400>  13
cttactcctt ggaggccatg tag                                               23


<210>  14
<211>  19
<212>  DNA
<213>  Artificial

<220>
<223>  Fw primer for SMS1

<400>  14
tacactgtgg acgtggtgg                                                    19

<210>  15
<211>  19
<212>  DNA
<213>  Artificial

<220>
<223>  Rv primer for SMS1

<400>  15
gggccaatgg taagatcga                                                    19


<210>  16
<211>  19
<212>  DNA
<213>  Artificial

<220>
<223>  Fw primer for SMS2

<400>  16
tcaatggaga ctctcaggc                                                    19


<210>  17
<211>  19
<212>  DNA
<213>  Artificial

<220>
<223>  Rv primer for SMS2

<400>  17
ccgctgaaga ggaagtctc                                                    19